(19) Europäisches Patentamt
European Patent Office
Office européen des brevets

(11) EP 0 536 371 B1

(12) # EUROPÄISCHE PATENTSCHRIFT

(45) Veröffentlichungstag und Bekanntmachung des Hinweises auf die Patenterteilung:
**10.07.1996 Patentblatt 1996/28**

(21) Anmeldenummer: **92909221.1**

(22) Anmeldetag: **22.04.1992**

(51) Int Cl.[6]: **C07H 15/203**, C12Q 1/40

(86) Internationale Anmeldenummer:
**PCT/EP92/00888**

(87) Internationale Veröffentlichungsnummer:
**WO 92/18515 (29.10.1992 Gazette 1992/27)**

(54) **INDOPHENOL-SUBSTITUIERTE MALTOOLIGOSIDE ALS alpha-AMYLASE-SUBSTRATE**

INDOPHENOL-SUBSTITUTED MALTO-OLIGOSIDES AS alpha-AMYLASE SUBSTRATES

MALTO-OLIGOSIDES SUBSTITUES PAR L'INDOPHENOL COMME SUBSTRATS DE L'alpha-AMYLASE

(84) Benannte Vertragsstaaten:
**AT BE CH DE DK ES FR GB GR IT LI LU NL PT SE**

(30) Priorität: **23.04.1991 DE 4113238**

(43) Veröffentlichungstag der Anmeldung:
**14.04.1993 Patentblatt 1993/15**

(73) Patentinhaber: **BOEHRINGER MANNHEIM GMBH**
**D-68298 Mannheim (DE)**

(72) Erfinder:
- **JUNIUS-COMER, Martina**
  **D-8127 Iffeldorf (DE)**
- **SCHMIDT, Axel**
  **D-8000 München 19 (DE)**
- **RAUSCHER, Elli**
  **D-8000 München 70 (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**81635 München (DE)**

(56) Entgegenhaltungen:
**EP-A- 0 263 435**

- **CHEMICAL ABSTRACTS, vol. 114, 1991, Columbus, Ohio, US; abstract no. 185925U, SHINOKI ET AL: 'Preparation of Indophenol alpha-Glycosides as Reagents for alpha-Amylase Analysis' Seite 836 ;**
- **CHEMICAL AND PHARMACEUTICAL BULLETIN. Bd. 38, Nr. 1, 1990, TOKYO JP Seiten 13 - 18; S. TOKUTAKE ET AL.: 'Glycosides Having Chromophores as Substrates for Sensitive Enzyme Analysis. 1.'**

Bemerkungen:
Verbunden mit 92106895.3/0510620 (europäische Anmeldenummer/Veröffentlichungsnummer) durch Entscheidung vom 11.04.96.

EP 0 536 371 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft Maltoside und Maltotrioside, die an ihrem reduzierenden Ende in α-Stellung mit einem substituierten Indophenolrest verknüpft sind und die Verwendung dieser Verbindungen als synthetische Enzymsubstrate zur direkten Bestimmung von α-Amylase, insbesondere zur Bestimmung von Pankreas-α-Amylase sowie ein dazu geeignetes Reagenz.

α-Amylase (E.C. 3.2.1.1) baut 1,4-α-glucosidisch verknüpfte Oligo- und Polysaccharide überwiegend durch Hydrolyse der 1,4-α-glucosidischen Bindungen zu Maltose und Maltooligosiden ab. Das Enzym hat neben der Verwendung bei der industriellen Fermentationstechnik erhebliche Bedeutung im Rahmen der klinischen Analytik und Diagnostik. Insbesondere hat die Bestimmung der Pankreas-α-Amylase erhebliche diagnostische Bedeutung, da bei zahlreichen Erkrankungen sich der Gehalt dieses Enzyms in Körperflüssigkeiten wie etwa Serum, Harn oder Duodenalsekret beträchtlich verändert.

Es sind eine Vielzahl von Verfahren zur Bestimmung der α-Amylase sowie dafür geeignete synthetische Enzymsubstrate bekannt.

Die EP-A 0346 912 offenbart Maltooligoside, die an ihrem reduzierenden Ende in α- oder β-Stellung mit einem Phenylrest verknüpft sind, der in Para-Position durch eine Nitro- oder Nitrovinylgruppe substituiert ist. Diese Maltooligoside werden als geeignete Substrate zur Bestimmung der Aktivität von Hydrolasen, Lipasen, Esterasen, α-Glucosidase oder Glucoamylase beschrieben.

Die EP-A 0 319 933 offenbart Maltooligoside, deren nicht reduzierendes Ende durch Schutzgruppen blockiert ist und die am reduzierenden Ende in α- oder β-Stellung mit substituierten oder nicht substituierten Phenylresten verknüpft sind. Beispiele für substituierte Phenylreste sind Halogen-, Hydroxyl-, Alkyl-, Alkoxy-, Alkoxycarbonyl-, Nitro- und Phenolsubstituierte Phenylreste. Besonders bevorzugt ist der Phenylrest durch eine oder mehrere Nitrogruppen substituiert. β-verknüpfte Maltooligoside des obigen Typs werden als geeignet zur Bestimmung von α-Amylase in Gegenwart von Hilfsenzymen wie etwa α- und β-Glucosidase bezeichnet. Eine direkte Bestimmung von α-Amylase ohne die Anwesenheit von Hilfsenzymen ist nicht offenbart.

Die japanische Offenlegungsschrift 64-42497 offenbart Maltooligoside, die an ihrem reduzierenden Ende in β-Stellung mit einem Indophenolrest der Formel

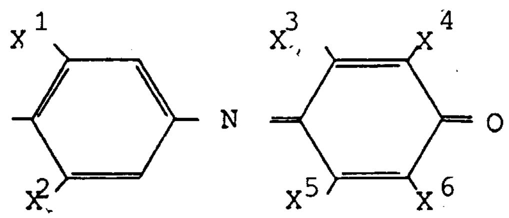

substituiert sind, wobei $x^1$ bis $X^6$ Wasserstoff- bzw. Halogen-Atome bzw. Nitro-, Cyan-, Azid-, Acyl-, Sulfonsäure-, Nitroso-, Sulfonyl-, Sulfoxyl-, Thiocyan-, Isothiocyan-, Isonitril-, Imino-, Azo-, Diazo-, Alkyl-, Allyl- bzw. Arylgruppen bedeuten, wobei $X^3$ und $X^4$ und/oder $X^5$ und $X^6$ verbunden sein und kondensierte aromatische Ringe bilden können.

Weiterhin werden Verfahren zur Herstellung dieser Verbindungen offenbart, sowie die Verwendung als α-Amylasesubstrate in Gegenwart von α- und β-Glucosidase. In den Beispielen werden konkret die Herstellung der Verbindungen Phenolindo-3'-chlorphenyl-β-maltopentaosid, Hexadecaacetyl-(phenolindo-3',5'-dichlorphenyl)-β-maltopentaosid, Phenolindo-3',5'-dichlorphenyl-β-maltoheptaosid, Hexadecaacetyl-(2,5-dimethylphenolindo-3'-chlorphenyl-β)-pentaosid und Hexadecaacetyl-(1-naphtholindo-3'-chlorphenyl)-β-pentaosid offenbart.

Die EP-A 0 263 435 offenbart aromatisch substituierte Glycoside der Formel

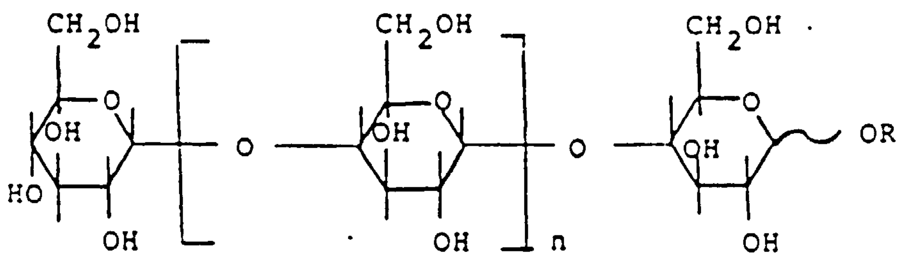

(I)

worin der Substituent R am reduzierenden Ende der Verbindung in α-Stellung vorliegt, n = 0 oder 1 ist und R ein substituierter aromatischer Rest ist, ausgewählt aus der Gruppe

(a) 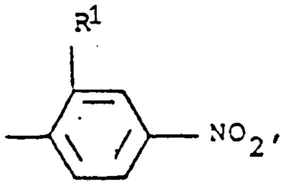 (b) 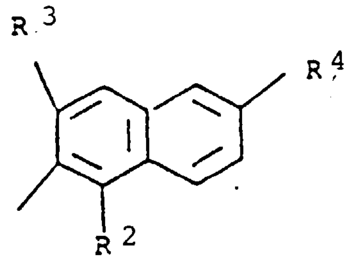 und (c) 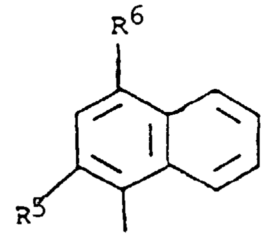

worin $R^1$ bis einschließlich $R^6$ unabhängig Halogen, $NO_2$, $SO_3H$,

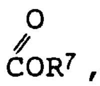, 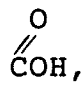, 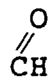 oder 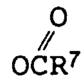

sind, wobei $R^7$ ein niederer Alkylrest ist, einschließlich seiner Stereoisomere, optischen Isomere und geometrischen Isomere und Mischungen dieser Isomere.

Diese Substrate sind als direkte Substrate (d.h. ohne Anwesenheit von Hilfsenzymen) für α-Amylasen beschrieben. In den Beispielen wird konkret die Herstellung von 2-Chlor-4-nitrophenyl-α-D-maltotriosid, 4-Chlor-2-nitro-1-naphthyl-α-maltotriosid und 2-Formyl-4-nitrophenyl-α-D-maltotriosid offenbart. Weiterhin wird ein Verfahren für die Herstellung der Substrate und verwandter Substanzen offenbart. Die in der EP-A 0 263 435 offenbarten Verbindungen weisen eine maximale Absorption bei Wellenlängen von ca. 385 bis 450 nm auf.

Die japanische Offenlegungsschrift 02/306990 beschreibt Indophenyl-α-glycoside mit 2 bis 10 Glucoseeinheiten, Indophenylresten als Chromophor und unsubstituierten OH-Gruppen am Oligoglucosid. Als Beispiel offenbart ist Phenolindo-3'-chlorphenyl-α-D-maltopentaosid, dessen Bestimmung in Gegenwart des Hilfsenzyms α-Glucosidase erfolgt.

Die japanische Offenlegungsschrift 02/306991 offenbart cyclische Acetale von Indophenylglucosiden mit α- oder β-Verknüpfung und 2 bis 10 Glucoseeinheiten, die Indophenylreste als Chromophor aufweisen und bei denen die OH-Gruppen in den Positionen $C_4$ und $C_6$ der Glucose am nicht reduzierenden Ende durch Alkyl- oder Alkylidenreste substituiert sind. Als bevorzugt werden Verbindungen mit 5 bis 7 Glucoseeinheiten bezeichnet. Als Beispiel ist Isopropylidenphenolindophenyl-α-maltopentaosid offenbart, dessen Bestimmung in Gegenwart der Hilfsenzyme α- und β-Glucosidase durchgeführt wird.

Die japanische Offenlegungsschrift 03/085996 offenbart Indophenyl-β-maltooligoside mit 3 bis 8 Glucoseeinheiten, die Indophenylresten als Chromophor und unsubstituierte oder acylsubstituierte OH-Gruppen aufweisen und bei denen die OH-Gruppen in den Positionen $C_4$ und $C_6$ der Glucose am nicht reduzierenden Ende gegebenenfalls mit Alkylidengruppen substituiert sind. Beispielhaft offenbart ist Isopropylidenphenolindophenyl-β-D-maltopentaosid, dessen Bestimmung in Gegenwart der Hilfsenzyme α- und β-Glucosidase erfolgt.

Alle oben genannten Verfahren und Enzymsubstrate für die Bestimmung von α-Amylase weisen jedoch Nachteile auf. So sind die oben genannten Verbindungen mit 5 oder mehr Glucoseeinheiten, bei denen der Substituent am reduzierenden Ende des Zuckers in α- oder β-Stellung verknüpft ist, nicht als direkte Substrate, sondern nur in Anwesenheit von Hilfsenzymen zur Bestimmung der α-Amylase geeignet. Andere Substanzen sind als Substrate ungeeignet, weil sie wenig sensitiv sind und ihre Absorptionsmaxima bei ungünstigen Wellenlängen liegen. So ist beispielsweise bekannt, daß bei Durchführung von α-Amylasetests mit Meßwellenlängen von unterhalb 500 nm Störungen auftreten können, die auf in Körperflüssigkeiten anwesende Verbindungen wie etwa Hämoglobin und Bilirubin zurückzuführen sind.

Eine Aufgabe der vorliegenden Erfindung war es daher, α-Amylasesubstrate bereitzustellen, die im langwelligen Bereich, d.h. oberhalb ca. 550 nm absorbieren und die direkt ohne Erfordernis von Hilfsenzymen von der α-Amylase gespalten werden können.

Die erfindungsgemäße Aufgabe wird gelöst durch Verbindungen der allgemeinen Formel I

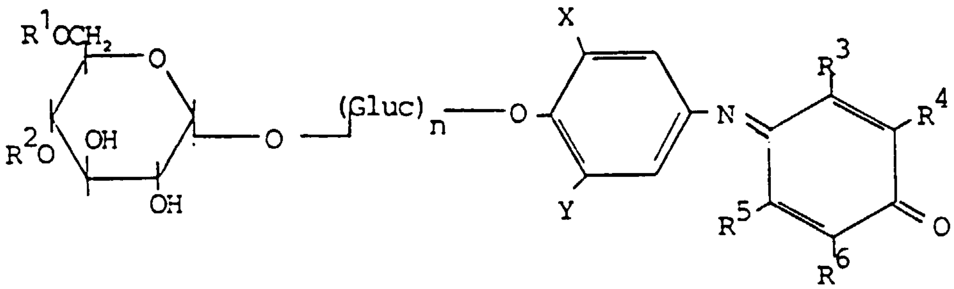

(I)

worin Gluc ein Glucosemolekül ist und der Indophenolrest in α-Stellung mit dem Zucker verknüpft ist,

n = 1 oder 2,
$R^1$ = H, eine geradkettige oder verzweigte Alkyl- oder Alkoylgruppe mit 1 bis 6 C-Atomen, eine Cycloalkyl- oder Cycloalkoylgruppe mit 3 bis 6 C-Atomen, eine Benzoyl-, Benzyl- oder Phenylgruppe,
$R^2$ = H,
oder worin $R^1$ und $R^2$ zusammen eine Methylenbrücke bilden, deren H-Atome unabhängig voneinander durch je eine Alkylgruppe mit 1 bis 5 C-Atomen oder eine Phenylgruppe substituiert sein können,
$R^3$, $R^4$, $R^5$, $R^6$ = unabhängig voneinander H, eine Halogen-(F, Cl, Br), Nitro-, Acetamid- oder Cyanogruppe, eine Sulfonsäure-, Sulfinsäure- oder Carbonsäuregrupe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe, wobei $R^3$ und $R^4$ oder/und $R^5$ und $R^6$ miteinander verbunden sein und kondensierte Ringe bilden können,
X, Y = unabhängig voneinander H, eine Halogen- (F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe, unter der Voraussetzung, daß X und Y nicht gleichzeitig H sind.

Die in den obigen Definitionen genannte Arylgruppe ist vorzugsweise Phenyl oder Naphthyl. Die Alkoxygruppe kann eine oder mehrere O-Brücken enthalten, sie kann daher z.B. auch eine Polyethylenglykolkette sein. Die Alkyl-, Cycloalkyl- und Arylreste sind gegebenenfalls substituiert, d.h. sie können Substituenten (wie z.B. Halogengruppen) tragen, die keine chemische Reaktion mit α-Amylase unter den beim enzymatischen Bestimmungstest herrschenden Bedingungen eingehen.

Überraschenderweise sind die Verbindungen der allgemeinen Formel I sehr gut als synthetische Enzymsubstrate für die α-Amylase geeignet, da sie direkt (d.h. in Abwesenheit von Hilfsenzymen) von der α-Amylase gespalten werden können und zugleich eine Absorption im langwelligen Bereich oberhalb ca. 550 nm zeigen. Die Verwendbarkeit der erfindungsgemäßen Substanzen ist umso überraschender, da Verbindungen mit analoger Struktur, bei denen aber an beiden Positionen X und Y des Phenylrings im Indophenolrest Wasserstoffatome vorhanden sind, von der α-Amylase nicht direkt gespalten werden können.

Die Substituenten $R^1$ und $R^2$ am nicht reduzierenden Ende der Verbindung I sind vorzugsweise Wasserstoffatome. Es ist jedoch auch möglich, daß die Verbindung I an diesen Positionen durch Substituenten blockiert ist. Die Einführung solcher Substituenten ist detailliert in der DE-39 29 355 beschrieben. Demgemäß kann die Einführung der Reste $R^1$ und $R^2$ aus den entsprechenden unsubstituierten Verbindungen I erfolgen, indem man diese unter Veretherungsbedingungen mit einer Dialkoxy-Verbindung, vorzugsweise Dialkoxyethan oder dem entsprechenden Benzylderivat umsetzt unter Bildung einer Verbindung der allgemeinen Formel I, in welcher $R^1$ und $R^2$ zusammen eine gegebenenfalls substituierte Methylengruppe bilden.

Es kann auch in das ungeschützte Substrat die gewünschte Substituentengruppe über aktivierte Carbonsäuregruppen wie z.B. über die entsprechenden Orthoester, Säurechloride, Anhydride, aus aktivierten Estern enzymatisch (J.A.C.S 110 (1988), 584-589), aus Acetalen oder direkt aus der Carbonsäure über wasserentziehende Mittel wie z. B. durch die Mitsunobu-Reaktion (Tetrahedron Letters Vol. 30 (1989), 325-326) eingeführt werden. Besonders bevorzugt ist die enzymatische Herstellung, die über die entsprechenden Orthoester als Zwischenprodukte verläuft und die Herstellung über die Mitsunobu-Reaktion. Die Orthoester werden bevorzugt aus den entsprechenden Nitrilen hergestellt (vgl. z.B. Houben-Weyl, Band VI/3 (1965), 300-313). Die Reinigung dieser geschützten Substrate kann z.B. chromatographisch über Ionenaustauscher oder HPLC erfolgen.

In der allgemeinen Formel I bedeutet n = 1 oder 2, daher handelt es sich bei den erfindungsgemäßen Verbindungen um Maltoside oder Maltotrioside. Höhere Maltooligoside werden von der α-Amylase als direkte Substrate nicht mehr

akzeptiert. Vorzugsweise handelt es sich bei den erfindungsgemäßen Verbindungen um Maltotrioside.

Die Reste X und Y am Phenylring des Indophenolrests von I können unabhängig voneinander H, eine F-, Cl- oder Br-, Acetamid-, Nitro- oder Cyanogruppe, eine Sulfonsäure-, Sulfinsäure- oder Carbonsäuregruppe sowie entsprechende Säurealkylester- und Säureamidgruppen, eine Alkoxygruppe (mit einer oder mehreren Sauerstoffbrücken, d. h. beispielsweise auch eine Polyethylenglycolkette), eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe sein, unter der Voraussetzung, daß X und Y nicht gleichzeitig H sind. Von den Verbindungen der Formel I sind allgemein diejenigen bevorzugt, bei denen einer der beiden Reste X und Y Wasserstoff und der andere einen von Wasserstoff verschiedenen Substituenten darstellt. Vorzugsweise ist auch mindestens eines von X und Y eine Halogengruppe, besonders bevorzugt ist X eine Halogengruppe und Y Wasserstoff. Am meisten bevorzugt ist es, wenn die Halogengruppe Chlor bedeutet.

Die Reste $R^3$, $R^4$, $R^5$ und $R^6$ in der allgemeinen Formel I stellen die möglichen Substituenten am Benzochinonring des Indophenolrests dar. Dabei können $R^3$, $R^4$, $R^5$ und $R^6$ unabhängig voneinander H, eine Halogen- (F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe sein, wobei $R^3$ und $R^4$ oder/und $R^5$ und $R^6$ miteinander verbunden sein und kondensierte Ringe bilden können, wobei die Alkoxygruppe eine oder mehrere O-Brücken aufweisen kann. Wenn die Reste $R^3$ und $R^4$ oder/und die Reste $R^5$ und $R^6$ miteinander verbunden sind, entstehen kondensierte Ringe. Auch bei einem solchen Substitutionsmuster sind die resultierenden Verbindungen I als direkte α-Amylase-Substrate geeignet.

$R^3$ und $R^5$ bedeuten vorzugsweise Wasserstoff und $R^4$ und $R^6$ bedeuten vorzugsweise Halogen, besonders bevorzugt Chlor. Am meisten bevorzugt von den Verbindungen der allgemeinen Formel I ist 2',2,6-Trichlor-indophenyl-α-maltotriosid, das aufgrund seiner langwelligen Absorption bei einer Meßwellenlänge von 645 nm bestimmt werden kann und hervorragend durch α-Amylase in Abwesenheit von Hilfsenzymen spaltbar ist.

Die Herstellung der erfindungsgemäßen Verbindungen erfolgt auf an sich bekannte Weise, wobei drei verschiedene Verfahren bevorzugt genannt werden können. Bei der ersten und bevorzugten Verfahrensvariante geht man von Maltose oder Maltotriose aus. Diese Verbindungen werden auf an sich bekannte Weise unter Natriumacetat-Katalyse peracetyliert (z.B. durch Umsetzung mit Essigsäureanhydrid), wobei eine Verbindung mit der allgemeinen Formel II erzeugt wird,

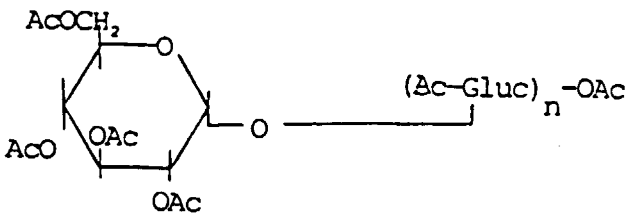

(II)

worin Ac-Gluc eine peracetylierte Glucose bedeutet und der Acetylrest am reduzierenden Ende von II in β-Stellung mit dem Zucker verknüpft ist.

Die Verbindung II wird anschließend in Gegenwart eines Säurekatalysators mit einem substituierten p-Nitrophenol der allgemeinen Formel III umgesetzt

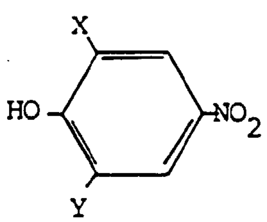

(III)

worin X und Y die oben genannten Bedeutungen besitzen. Als Säurekatalysator für diese Umsetzung sind z.B. Alkyl- oder Arylsulfonsäuren wie etwa p-Toluolsulfonsäure oder vorzugsweise Lewis-Säurekatalysatoren wie etwa $BF_3 \cdot OEt_2$ geeignet. Diese Reaktion ist eine stereospezifische Substitution des Acetylrests am reduzierenden Ende des Zuckers durch das p-Nitrophenol-III, die formal nach einem $S_N2$-Mechanismus verläuft, so daß in der resultierenden Verbindung IV

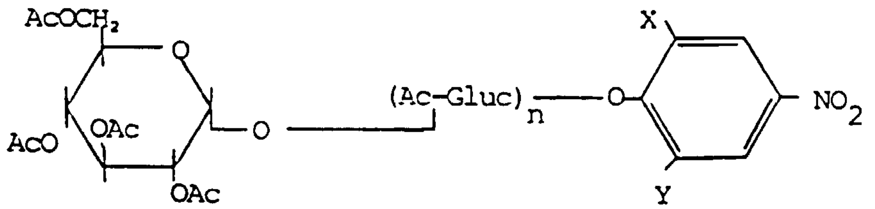

(IV)

der p-Nitrophenylrest in α-Stellung mit dem Zucker verknüpft ist. Anschließend wird die Nitrogruppe von IV auf an sich bekannte Weise (z.B. durch katalytische Hydrierung in Gegenwart von Pd-C) zu einer Aminogruppe reduziert. Danach werden die Acetylreste aus der resultierenden Verbindung z.B. durch alkalische Verseifung in Gegenwart von Ammoniak abgespalten.

Das deacetylierte Produkt wird schließlich mit einem gegebenenfalls substituierten p-Benzochinon der allgemeinen Formel V umgesetzt,

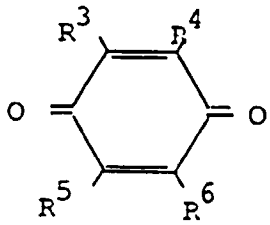

(V)

worin $R^3$, $R^4$, $R^5$ und $R^6$ die oben genannten Bedeutungen besitzen. Auf diese Weise wird eine Verbindung mit der allgemeinen Formel VI erzeugt,

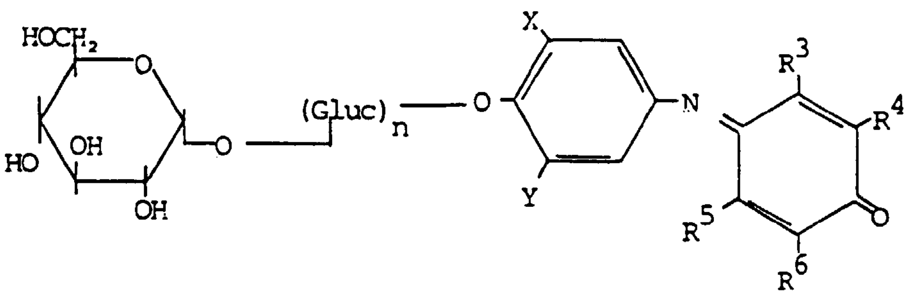

(VI)

worin der Indophenolrest in α-Stellung mit dem Zucker verknüpft ist. Diese Umsetzung findet vorzugsweise in einem wasserfreien aprotischen Lösungsmittel, wie etwa Dimethylformamid oder Dioxan oder einem Gemisch davon, in Gegenwart von Trifluoressigsäure statt.

Gegebenenfalls kann in einem weiteren Schritt eine Substitution der Wasserstoffatome an den Positionen 4 und 6 des Glucoserests am nicht reduzierenden Ende des Zuckers mit den Resten $R^1$ und $R^2$ erfolgen, wie bereits oben ausführlich erläutert wurde.

Alternativ dazu kann die Herstellung der Verbindung der allgemeinen Formel I dadurch erfolgen, daß man eine peracetylierte β-Maltose oder β-Maltotriose mit einem Indophenol der allgemeinen Formel VII umsetzt,

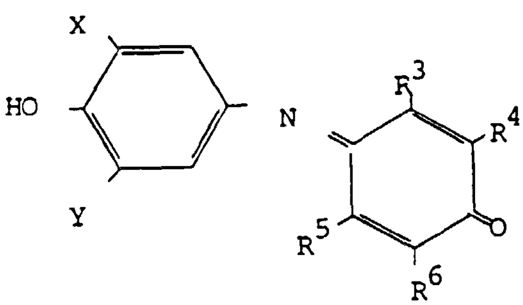

(VII)

worin X, Y, $R^3$, $R^4$, $R^5$ und $R^6$ die oben genannten Bedeutungen besitzen. Die weitere Aufarbeitung des resultierenden Produkts, d.h. Deacetylierung und gegebenenfalls Derivatisierung am nicht reduzierenden Ende, erfolgt analog der ersten Verfahrensvariante.

Eine dritte Möglichkeit zur Herstellung der erfindungsgemäßen Verbindungen ist es, wenn man nach einer der oben beschriebenen Verfahrensweisen zunächst ein Indophenyl-α-glucosid-Derivat der Formel VIII herstellt,

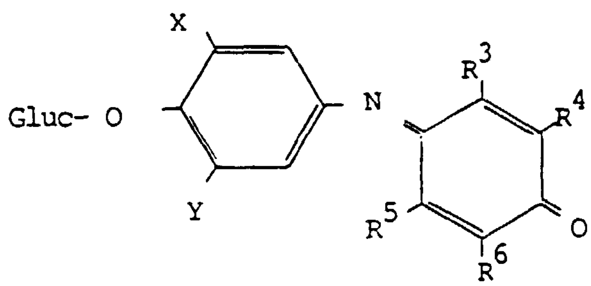

(VIII)

worin die jeweiligen Reste die oben genannten Bedeutungen besitzen und diese Verbindung anschließend in Gegenwart von Cyclodextrin, Amylose oder Stärke in Gegenwart von Cyclodextringlucosyltransferase durch "trimming" zu den erfindungsgemäßen Maltosid- und Maltotriosid-Derivaten umsetzt.

Verfahren zur Herstellung von β-Maltooligosidderivaten, die sich von den erfindungsgemäßen Verbindungen durch die Stellung des Substituenten am reduzierenden Ende des Zuckers unterscheiden, sind in der japanischen Offenlegungsschrift 64-42497 detailliert für die entsprechenden Indophenyl-β-Glucosidderivate offenbart. Die Herstellung der erfindungsgemäßen α-Maltooligosidderivate unterscheidet sich prinzipiell nur dadurch, daß man von einem peracetylierten β-Maltooligosidderivat ausgeht, während man zur Herstellung der β-Verbindungen von einem peracetylierten α-Maltooligosidderivat ausgeht.

Die Herstellung von α-Maltooligosidderivaten, die sich von den erfindungsgemäßen Substanzen durch die Art des Substituenten unterscheiden, ist in der EP-A 0 263 435 offenbart.

Ein weiterer Gegenstand der vorliegenden Erfindung ist ein Verfahren zur Bestimmung der Aktivität von α-Amylase in wäßriger Lösung, insbesondere in Körperflüssigkeiten mit einem synthetischen Enzymsubstrat, wobei man als Enzymsubstrat eine Verbindung mit der allgemeinen Formel I nach Anspruch 1 verwendet. Vorzugsweise verwendet man bei diesem Verfahren eine Verbindung I, in der $R^1$ und $R^2$ Wasserstoff bedeuten oder zusammen eine Methylenbrücke bilden, deren H-Atome unabhängig voneinander durch je eine Alkylgruppe mit 1 bis 5 C-Atomen oder eine Phenylgruppe substituiert sein können. Weiterhin ist bevorzugt, daß einer der Substituenten X und Y am Phenylring des Indophenolrests Wasserstoff ist und der andere Substituent von Wasserstoff verschieden ist. Weiterhin ist bevorzugt, wenn mindestens einer der Reste X und Y eine Halogengruppe bedeutet. Besonders bevorzugt ist die Verwendung von 2',2,6-Trichlor-indophenyl-a-maltotriosid als Enzymsubstrat.

Mit dem erfindungsgemäßen Verfahren läßt sich eine direkte Aktivitätsbestimmung von α-Amylase, d.h. eine Aktivitätsbestimmung in Abwesenheit von Hilfsenzymen wie etwa α-Glucosidase oder β-Glucosidase durchführen.

Ferner ist es bei dem erfindungsgemäßen Verfahren möglich, spezifisch die Aktivität von Pankreas-α-Amylase in Gegenwart von Speichel-α-Amylase unter Verwendung eines spezifischen Hemmstoffsystems für Speichel-α-Amylase durchzuführen. Ein solches Hemmstoffsystem, das einen oder mehrere monoklonale Antikörper umfaßt, wird z.B. in der DE 39 29 355 offenbart. Geeignete Antikörper sind z.B. in der EP-A 0 191 284, der EP-A 0 150 309 und in der EP-A 0 209 154 offenbart. Durch Kombination des erfindungsgemäßen Enzymsubstrats und des Hemmstoffs erfolgt die Bestimmung der Pankreas-α-Amylase sehr selektiv und sehr genau.

Weiterhin betrifft die vorliegende Erfindung ein Reagenz zur direkten spezifischen Bestimmung von α-Amylase mit einem synthetischen Enyzmsubstrat, das als Enzymsubstrat eine Verbindung der allgemeinen Formel I enthält. Vorzugsweise enthält das erfindungsgemäße Reagenz als Enzymsubstrat eine Verbindung I, in der $R^1$ und $R^2$ Wasserstoff bedeuten oder zusammen eine Methylenbrücke, deren H-Atome unabhängig voneinander durch je eine Alkylgruppe mit 1 bis 5 C-Atomen oder eine Phenylgruppe substituiert sein können. Weiterhin ist bevorzugt, daß in dem verwendeten Substrat der Formel I einer der Substituenten X und Y Wasserstoff ist und der andere Substituent von Wasserstoff verschieden ist. Weiterhin ist bevorzugt, wenn mindestens einer der Reste X und Y eine Halogengruppe bedeutet. Besonders bevorzugt ist ein erfindungsgemäßes Reagenz, das als Substrat 2',2,6-Trichlor-indophenyl-α-maltotriosid enthält. Die Endkonzentration des erfindungsgemäßen Substrats in der Enzymtestlösung beträgt vorzugsweise 1 bis 10 mmol/l, besonders bevorzugt 3 bis 7 mmol/l. Das erfindungsgemäße Reagenz kann auch zusätzlich einen Aktivator für die α-Amylase enthalten, z.B. ein Azid- oder Rhodanidsalz. Der Aktivator wird vorzugsweise mit einer Endkonzentration von 50 bis 700 mmol, besonders bevorzugt von 100 bis 500 mmol/l eingesetzt. Weiterhin kann das erfindungsgemäße Reagenz auch ein spezifisches Hemmstoffsystem für Speichel-α-Amylase enthalten, das aus einem oder mehreren monoklonalen Antikörpern besteht. Auf diese Weise ist eine selektive Bestimmung von Pankreas-α-Amylase möglich.

Die Erfindung betrifft weiterhin die Verwendung einer Verbindung der allgemeinen Formel I als synthetisches Enzymsubstrat, insbesondere zur direkten Bestimmung der enzymatischen Aktivität von α-Amylase.

Die Erfindung wird weiter durch die folgenden Beispiele in Verbindung mit der Zeichnung erläutert.

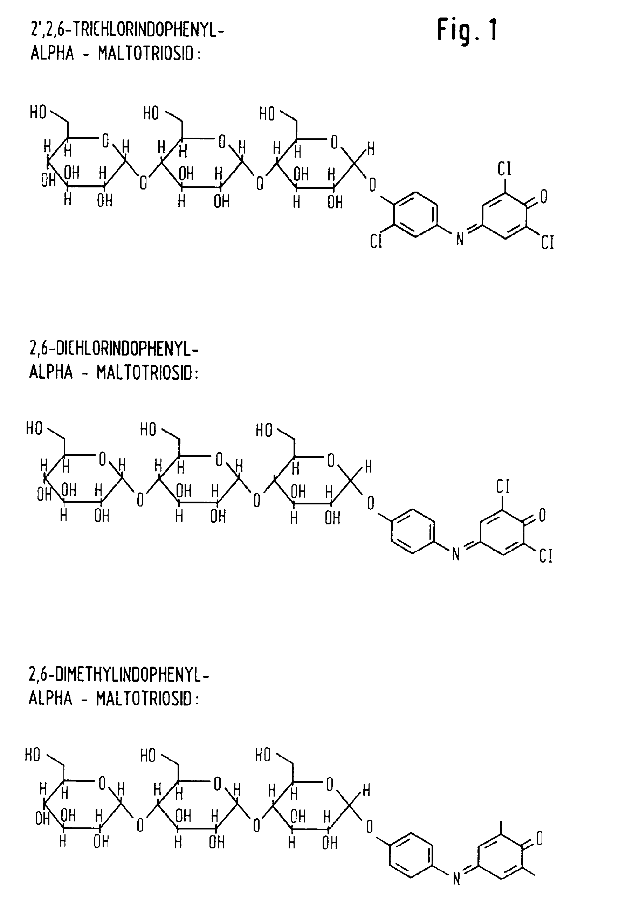

In der Zeichnung zeigt Fig. 1 die chemischen Strukturformeln der in Beispiel 2 getesteten α-Amylasesubstrate.

Beispiel 1

Synthese von 2',2,6-Trichlor-indophenyl-α-maltotriosid

**a) Deca-acetyl-(2-chlor-4-nitrophenyl)-α-maltotriosid**

0,1 mol peracetylierte β-Maltotriose, 0,3 mol 2-Chlor-4-nitrophenol und 25 ml $BF_3{}^\bullet OEt_2$ werden in einem Gemisch von 450 ml wasserfreiem Toluol und 100 ml wasserfreiem Dichlorethan 32 Stunden bei 45°C unter Feuchtigkeitsausschluß gerührt. Anschließend wird 500 ml Dichlormethan zugegeben und mit 800 ml gesättigter $Na_2\ CO_3$ -Lösung ausgeschüttelt. Die organische Phase wird nach Trocknung über $MgSO_4$ eingeengt. Der Rückstand wird über Kieselgel chromatographiert (Laufmittel: Essigester/Hexan 2:1).
Ausbeute: 27 g (25 %)
DC: $R_f$ = 0,59 (Kieselgel; Toluol/Aceton 7/4)

**b) Deca-acetyl-(2-chlor-4-aminophenyl)-α-maltotriosid**

6,5 g (6 mmol) des Produktes aus 1a) werden in einem Gemisch von 160 ml Dioxan, 60 ml Methanol, 2 ml Eisessig gelöst und mit 0,65 g 10 %igem Pd-C versetzt. Die Hydrierung wird 7 Stunden unter einem Druck von 0,9 bar durchgeführt. Nach Abfiltrieren des Katalysators und Entfernen des Lösungsmittels wird der Rückstand über Kieselgelchromatographie (Laufmittel: Toluol/Aceton 7:3) gereinigt.
Ausbeute: 1,6 g (25 %)
DC: $R_f$ = 0,42 (Kieselgel; Toluol/Aceton 7/4)

**c) 2-Chlor-4-aminophenyl-α-maltotriosid**

1 g (1 mmol) des Produkts aus 1b) werden in 50 ml Methanol, 5 ml Wasser und 10 ml 25 % Ammoniak versetzt und 20 Stunden bei Raumtemperatur gerührt. Das Lösungsmittel wird abgezogen und das Produkt ohne weitere Aufreinigung weiter umgesetzt. Ausbeute: 680 mg
DC: $R_f$ = 0,47 (Kieselgel; Butanol/Eisessig/Wasser 10/3/5)

**d) 2',2,6-Trichlor-indophenyl-α-maltotriosid**

680 mg des Produkts aus 1c) werden in 10 ml wasserfreiem Dimethylformamid, 30 ml wasserfreiem Dioxan, 0,2 ml Trifluoressigsäure gelöst und mit 1 g (5,8 mmol) 2,6-Dichlor-benzochinon und 3 Spatelspitzen Molekularsieb versetzt. Das Gemisch wird 2 Stunden unter Feuchtigkeitsausschluß bei Raumtemperatur gerührt. Anschließend wird filtriert, eingeengt, der Rückstand mit Wasser aufgenommen und nochmals filtriert. Das Filtrat wird über eine Gelchromatographie (LH 20, Laufmittel: Wasser) vorbehandelt. Abschließend wird über präparative HPLC endgereinigt (RP 18; Gradient: Wasser/Isopropanol; 0,1 % Trifluoressigsäure: 80/20 % → 65/35 %).
Ausbeute: 60 mg (8 %)
DC: $R_f$ = 0,62 (Kieselgel; Butanol/Eisessig/Wasser 10/3/5)

Beispiel 2

Verwendung von 2',2,6-Trichlor-indophenyl-α-maltotriosid als Substrat zur direkten α-Amylase-Bestimmung

Es wurde mit den in der folgenden Tabelle und in Fig. 1 aufgeführten Substraten, die Amylaseaktivität von Human-Pankreas-α-Amylase (HPA) und Human-Speichel-α-Amylase (HSA) untersucht. Die folgenden Reagenzien wurden verwendet:

Reagenz 1:

| | | |
|---|---|---|
| 4-Morpholinethansulfonat (=MES)-Puffer | | 61,6 mMol/l pH 6,0 |
| enthaltend | Nacl | 57,1 mMol/l |
| | Calciumacetat | 5,6 mMol/l |

Reagenz 2:

| | |
|---|---|
| Substrat (wie in der Tabelle angegeben) | 49,3 m/Mol/l |

Reagenz 1 enthält gegebenenfalls noch Aktivator, wie in der Tabelle angegeben, sowie - als Hemmstoffe für die Speichel-α-Amylase- von den Zellinien NCACC 84122003 (MAK I) und NCACC 84111301 (MAK II) produzierte Antikörper. Zur Durchführung der Bestimmung wird 1,00 ml Reagenz 1 mit 0,02 ml der Probe gemischt und auf 37°C temperiert. Anschließend wird 0,10 ml Reagenz 2 zugegeben, gemischt und nach 1,2 und 3 Minuten die Extinktion bei der jeweiligen Meßwellenlänge (s. Tabelle) abgelesen. Es wird der Mittelwert der Extinktionsdifferenz/Minute gebildet.

Berechnung:

$$\text{U/L Probe} = \frac{\Delta E/\text{min} \times 1{,}12 \times 1000}{\varepsilon \times 0{,}02}$$

Die Endkonzentrationen im Testansatz betragen:

| | |
|---|---|
| MES-Puffer, pH 6,0 | 55 mmol/l |
| NaCl | 51 mmol/l |
| Calciumacetat | 5 mmol/l |
| Substrat gegebenenfalls Aktivator | 4,44 mmol/l |
| Natriumazid | 152 mmol/l |
| bzw. Kaliumthiocyanat | 400 mmol/l |
| MAK I | ca. 5 bis 8 mg/l |
| MAK II | ca. 2 bis 3 mg/l |

Als Probe wird Humanpankreas-Amylase (HPA) verwendet. Die Messung erfolgt bei 37°C bei der jeweils angegebenen Meßwellenlänge.

Die Ergebnisse sind der Tabelle zu entnehmen.

Tabelle

| Substrat | Meßwellenlänge [nm] | 400 mMol/l KSCN HPA [ΔE/min] |
|---|---|---|
| 2',2,6-Trichlor-indophenyl-α-maltotriosid | 645 | 0,063 |
| 2,6-Dimethyl-indophenyl-α-maltotriosid | 578 | 0* |
| 2,6-Dichlor-indophenyl-α-maltotriosid | 578 | 0 |

* Mit 150 mmol/l $NaN_3$ anstatt KSCN

Die erhaltenen Ergebnisse zeigen, daß nur Indophenylderivate, die an Position 2' des Phenylringes einen von Wasserstoff verschiedenen Substituenten, insbesondere einen Halogensubstituenten besitzen, direkte Amylase-Substrate darstellen. Fehlt der Substituent in der 2-Position, tritt keine Amylase-Spaltung ein.

Beispiel 3

**a) Isopropyliden-2-chlor-4-nitrophenyl-α-D-maltotriosid**

14,25 g Deca-acetyl-(2-chlor-4-nitrophenyl)-α-maltotriosid aus Beispiel la) werden in 450 ml Methanol gelöst. Dazu gibt man 45 ml Wasser und 90 ml konz. wäßrige Ammoniaklösung und rührt 16 Stunden bei Raumtemperatur. Man engt ein und löst 660 mg (1 mmol) des erhaltenen 2-Cl-4-Nitrophenyl-α-D-maltotriosids in 15 ml DMF. Nacheinander werden 1,25 ml (10 mmol) 2,2-Dimethoxypropan (Merck 802936) und 172 mg (1 mmol) p-Toluolsulfonsäure, wasserfrei, zugegeben. Bei 48°C läßt man unter Rühren 80 Min reagieren. Dann wird der Ansatz in 15 ml 0,24 mol/l $NaHCO_3$-Lsg getropft. Das Gemisch wird 2 mal mit je 40 ml Essigsäureethylester geschüttelt. Die Wasserphase wird eingeengt und anschließend auf einer Sephadex LH20-Säule (Pharmacia) chromatographiert. Eluent: Wasser.

Die geeigneten Fraktionen werden lyophilisiert.

Ausbeute: 150 mg

DC: Rf = 0,51 (Kieselgel-DC, Merck 5735,

Fließmittel 1-Butanol/Eisessig/Wasser = 10/3/5)

**b) Isopropyliden-2-chlor-4-aminophenyl-α-D-maltotriosid**

150 mg Isopropyliden-2-chlor-4-nitrophenyl-α-D-maltotriosid aus Beispiel 3a) werden in 10 ml Methanol gelöst und mit 25 ml Tetrahydrofuran und 30 mg Pd auf Aktivkohle (10 % Pd, Merck 807104) versetzt.

Man hydriert 2 Stunden unter heftigem Schütteln ohne Überdruck. Der gesamte Ansatz wird vom Katalysator filtriert, eingedampft und nach 30 Min. an der Hochvakuumpumpe getrocknet.
Ausbeute: 140 mg
DC: Rf = 0,44 (Kieselgel, Merck 5735.
Fließmittel 1-Butanol/Eisessig/Wasser = 10/3/5)

**c) Isopropyliden-2',2,6-trichlorindophenyl-α-D-maltotriosid**

140 mg (0,2 mmol) Isopropyliden-2-chlor-4-aminophenyl-α-D-maltotriosid aus Beispiel 3b) werden in 1,5 ml Dimethylformamid, destilliert und wasserfrei, gelöst. Dazu werden 3 ml Dioxan, wasserfrei, und 3 Tropfen $BF_3$-Ethyletherkomplex gegeben. Man kühlt auf 10°C ab und gibt 0,22 g (1,2 mmol) 2,6-Dichlorbenzochinon zu. Man rührt 15 Min. bei 10°C, läßt dann auf Raumtemperatur erwärmen und rührt noch weitere 60 Min.

Zu dem Reaktionsansatz werden 40 ml Diethylether gegeben, worauf das Produkt ausflockt. Man filtriert ab und wäscht den Rückstand mit Diethylether. Das Produkt wird durch Aufreinigung an Sephadex LH 20 (Pharmacia) mit Wasser als Eluent erhalten.
DC: Rf = 0,60 (Kieselgel, Merck 5735,
Fließmittel 1-Butanol/ Eisessig/Wasser = 10/3/5)

Beispiel 4

Synthese von 2'-Chlor-2-methyl-indophenyl-α-maltotriosid

3,5 g des Produkts aus 1c) werden in 15 ml wasserfreiem Dimethylformamid, 60 ml wasserfreiem Dioxan, 1,3 ml $BF_3$-Ethyletherkomplex gelöst und mit 2,45 g (20 mmol) 2-Methyl-4-benzochinon und 1 Spatellöffel Molekularsieb versetzt. Das Gemisch wird über Nacht (16 Std.) bei Raumtemperatur und unter Feuchtigkeitsausschluß gerührt. Anschließend werden 200 ml Essigsäureethylester zugegeben und der hieraus entstandene Niederschlag über eine G4-Fritte abgesaugt. Der Rückstand wird in Wasser gelöst und über eine Sephadex LH 20-Säule gereinigt.
Ausbeute: 230 mg
DC: Rf = 0,44 (Kieselgel;
1-Butanol/Eisessig/Wasser 10/3/5)

Beispiel 5

Synthese von 2'-Chlor-2,6-dimethyl-indophenyl-α-maltotriosid

500 mg des Produkts aus 1c) werden in 5 ml wasserfreiem Dimethylformamid, 10 ml wasserfreiem Dioxan, 0,2 ml $BF_3$-Ethyletherkomplex gelöst und mit 0,55 g ( 3 mmol) 2,6-Dimethyl-4-benzochinon und 2 Spatelspitzen Molekularsieb versetzt. Das Gemisch wird über Nacht bei Raumtemperatur und unter Feuchtigkeitsausschluß gerührt. Anschließend werden 20 ml Essigsäureethylester und 20 ml Diethylether zugegeben und der entstandene Niederschlag über eine G4-Fritte abgesaugt. Dieser orange-rote Rückstand wird in Wasser gelöst und über eine Sephadex LH 20-Säule gereinigt.
Ausbeute: 35 mg
DC: Rf = 0,44 (Kieselgel;
1-Butanol/Eisessig/Wasser 10/3/5)

Beispiel 6

Verwendung der gemäß Beispiel 4 und Beispiel 5 hergestellten Substanzen als Substrate zur direkten α-Amylase-Bestimmung.

Es wird analog zu Beispiel 2 vorgegangen.

Die Ergebnisse sind der Tabelle 2 zu entnehmen.

Tabelle 2

| Substrat | Meßwellenlänge [nm] | 400 mmol/l KSCN HPA relativer Wert [ΔE/min] |
|---|---|---|
| 2',2,6-Trichlor-indophenyl-α-maltotriosid (Beispiel 1) | 645 | 1 |
| 2'-Chlor-2-methylindophenyl-α-maltotriosid (Beispiel 4) | 595 | 0,35* |
| 2'-Chlor-2,6-dimethyl-indophenyl-α-maltotriosid (Beispiel 5) | 573 | 0,27* |

* bei pH 8,0 gemessen

## Patentansprüche

**Patentansprüche für folgende Vertragsstaaten : AT, BE, CH, DE, DK, FR, GB, IT, LI, LU, NL, SE**

1. Verbindung der allgemeinen Formel (I)

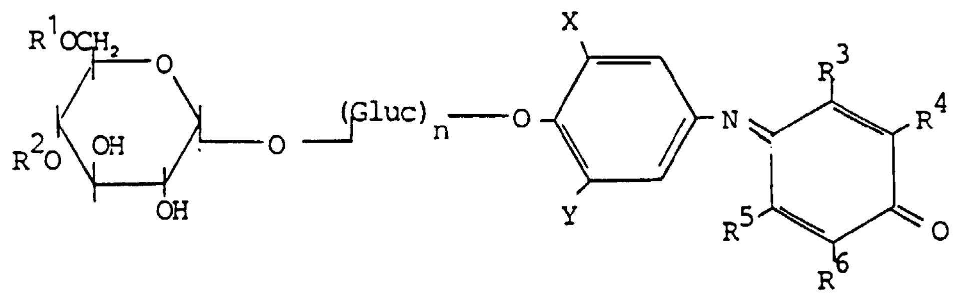

(I)

worin Gluc ein Glucosemolekül ist und der Indophenolrest in α-Stellung mit dem Zucker verknüpft ist,

n = 1 oder 2,
$R^1$ = H, eine geradkettige oder verzweigte Alkyl- oder Alkoylgruppe mit 1 bis 6 C-Atomen, eine Cycloalkyl- oder Cycloalkoylgruppe mit 3 bis 6 C-Atomen, eine Benzoyl-, Benzyl- oder Phenylgruppe,
$R^2$ = H,
oder worin $R^1$ und $R^2$ zusammen eine Methylenbrücke bilden, deren H-Atome unabhängig voneinander durch je eine Alkylgruppe mit 1 bis 5 C-Atomen oder eine Phenylgruppe substituiert sein können,
$R^3$, $R^4$, $R^5$, $R^6$ = unabhängig voneinander H, eine Halogen-(F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe, wobei $R^3$ und $R^4$ oder/und $R^5$ und $R^6$ miteinander verbunden sein und kondensierte Ringe bilden können,
X, Y = unabhängig voneinander H, eine Halogen- (F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe,

unter der Voraussetzung, daß X und Y nicht gleichzeitig H sind.

2. Verbindung nach Anspruch 1,
**dadurch gekennzeichnet,** daß $R^1$ und $R^2$ Wasserstoff bedeuten.

3. Verbindung nach Anspruch 1 oder 2,
**dadurch gekennzeichnet,** daß n = 2 bedeutet.

**4.** Verbindung nach einem der Ansprüche 1 bis 3,
**dadurch gekennzeichnet,** daß einer der beiden Reste X und Y Wasserstoff und der andere von Wasserstoff verschieden ist.

**5.** Verbindung nach einem der Ansprüche 1 bis 4,
**dadurch gekennzeichnet,** daß mindestens einer der Reste X und Y eine Halogengruppe bedeutet.

**6.** Verbindung nach Anspruch 5,
**dadurch gekennzeichnet,** daß die Halogengruppe ein Chlor ist.

**7.** Verbindung nach einem der Ansprüche 1 bis 6,
**dadurch gekennzeichnet,** daß $R^3$ und $R^5$ Wasserstoff bedeuten und $R^4$ und $R^6$ Halogen bedeuten.

**8.** Verbindung nach Anspruch 7,
**dadurch gekennzeichnet,** daß $R^4$ und $R^6$ Chlor bedeuten.

**9.** 2',2,6-Trichlor-indophenyl-α-maltotriosid.

**10.** Verfahren zur Herstellung einer Verbindung mit der allgemeinen Formel I nach Anspruch 1,
**dadurch gekennzeichnet,**
daß man

(a) Maltose oder Maltotriose auf an sich bekannte Weise peracetyliert, wobei eine Verbindung mit der allgemeinen Formel II erzeugt wird

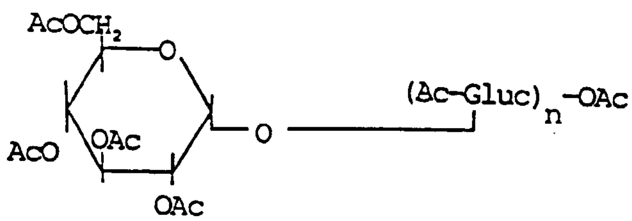

(II)

worin Ac-Gluc eine peracetylierte Glucose bedeutet und der Acetylrest am reduzierenden Ende von II in β-Stellung mit dem Zucker verknüpft ist,
(b) die Verbindung II in Gegenwart eines Säure-Katalysators mit einem substituierten p-Nitrophenol der allgemeinen Formel III umsetzt

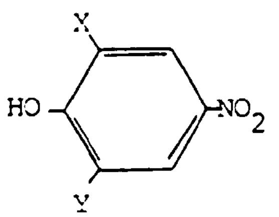

(III)

worin X und Y die in Anspruch 1 genannten Bedeutungen besitzen, wodurch eine Verbindung mit der allgemeinen Formel IV erzeugt wird,

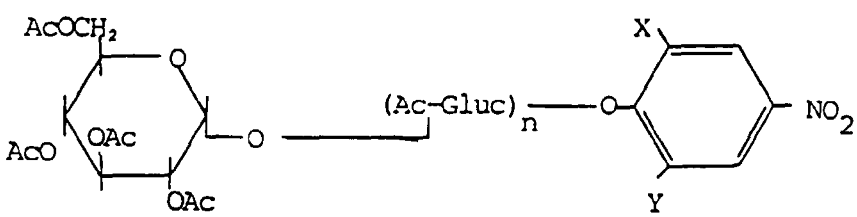

(IV)

worin der p-Nitrophenylrest in α-Stellung mit dem Zucker verknüpft ist,
(c) die Nitrogruppe von IV auf an sich bekannte Weise zu einer Aminogruppe reduziert,
(d) die Acetylreste der aus Schritt (c) resultierenden Verbindung abspaltet,
(e) die aus Schritt (d) resultierende Verbindung mit einem gegebenenfalls substituierten p-Benzochinon der

allgemeinen Formel V umsetzt,

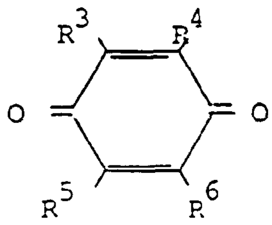

(V)

worin $R^3$, $R^4$, $R^5$ und $R^6$ die in Anspruch 1 genannten Bedeutungen besitzen, wobei eine Verbindung mit der allgemeinen Formel VI

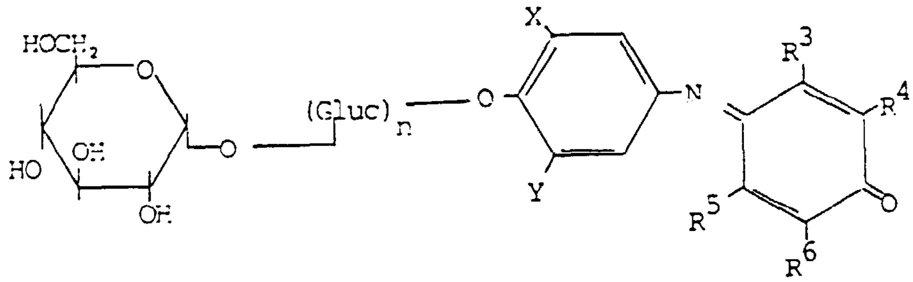

(VI)

worin der Indophenylrest in α-Stellung mit dem Zucker verknüpft ist, und gegebenenfalls

(f) auf an sich bekannte Weise eine Substitution der Positionen 4 und 6 am nicht reduzierenden Ende des Maltose- bzw. Maltotrioserests mit den Resten $R^1$ und $R^2$ einführt, wobei eine Verbindung mit der allgemeinen Formel I erzeugt wird,

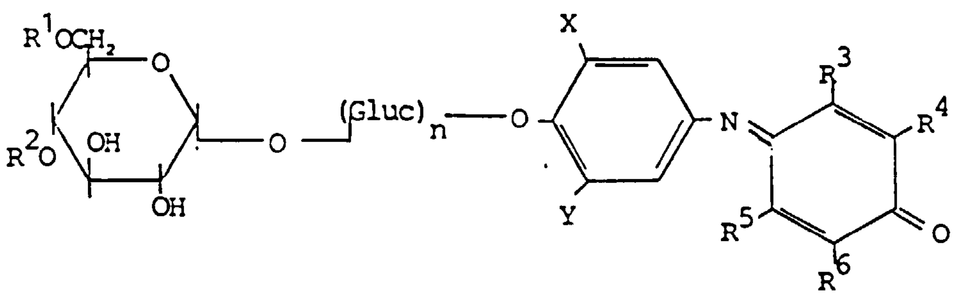

(I)

worin alle Symbole die in Anspruch 1 genannten Bedeutungen besitzen.

**11.** Verfahren zur Bestimmung der Aktivität von α-Amylase in wäßriger Lösung, insbesondere in Körperflüssigkeiten mit einem synthetischen Enzymsubstrat,
**dadurch gekennzeichnet,** daß man als Enzymsubstrat eine Verbindung mit der allgemeinen Formel I nach Anspruch 1 verwendet.

**12.** Verfahren nach Anspruch 11,
**dadurch gekennzeichnet,** daß man eine Verbindung I verwendet, in der $R^1$ und $R^2$ Wasserstoff bedeuten.

**13.** Verfahren nach Anspruch 11 oder 12,
**dadurch gekennzeichnet,** daß man eine Verbindung I verwendet, in der einer der beiden Reste X und Y Wasserstoff und der andere von Wasserstoff verschieden ist.

**14.** Verfahren nach Anspruch 13,
**dadurch gekennzeichnet,** daß man 2',2,6-Trichlor-indophenyl-α-maltotriosid als Enzymsubstrat verwendet.

**15.** Verfahren nach einem der Ansprüche 11 bis 14,
**dadurch gekennzeichnet,** daß man die Aktivitätsbestimmung von α-Amylase in Abwesenheit von Hilfsenzymen durchführt.

**16.** Verfahren nach einem der Ansprüche 11 bis 15,
**dadurch gekennzeichnet,** daß man spezifisch die Aktivität von Pankreas-α-Amylase in Gegenwart von Speichel-α-Amylase unter Verwendung eines spezifischen Hemmstoff-Systems für Speichel-α-Amylase bestimmt.

**17.** Verfahren nach Anspruch 16, **dadurch gekennzeichnet,** daß man als Hemmstoffsystem einen oder mehrere monoklonale Antikörper verwendet.

**18.** Reagenz zur direkten spezifischen Bestimmung von α-Amylase mit einem synthetischen Enzymsubstrat, **dadurch gekennzeichnet,** daß es als Enzymsubstrat eine Verbindung der allgemeinen Formel I nach Anspruch 1 enthält.

**19.** Reagenz nach Anspruch 18, **dadurch gekennzeichnet,** daß es als Substrat eine Verbindung I enthält, in der $R^1$ und $R^2$ Wasserstoff bedeuten.

**20.** Reagenz nach Anspruch 18 oder 19, **dadurch gekennzeichnet,** daß es als Substrat eine Verbindung I enthält, in der einer der beiden Reste X und Y Wasserstoff und der andere von Wasserstoff verschieden ist.

**21.** Reagenz nach Anspruch 20, **dadurch gekennzeichnet,** daß es als Substrat 2',2,6-Trichlor-indophenyl-α-maltotriosid enthält.

**22.** Reagenz nach einem der Ansprüche 18 bis 21, **dadurch gekennzeichnet,** daß es zusätzlich einen Aktivator enthält.

**23.** Reagenz nach Anspruch 22, **dadurch gekennzeichnet,** daß der Aktivator ein Azid- oder ein Rhodanidsalz ist.

**24.** Reagenz nach einem der Ansprüche 18 bis 23, **dadurch gekennzeichnet,** daß es ein spezifisches Hemmstoff-System für Speichel-α-Amylase enthält.

**25.** Reagenz nach Anspruch 24, **dadurch gekennzeichnet,** daß das Hemmstoff-System aus einem oder mehreren monoklonalen Antikörpern besteht.

**26.** Verwendung einer Verbindung nach einem der Ansprüche 1 bis 9 als synthetisches Enzymsubstrat.

**27.** Verwendung nach Anspruch 26 zur direkten Bestimmung der enzymatischen Aktivität von α-Amylase.

**Patenstansprüche für folgende Vertragsstaaten : ES, GB**

**1.** Verfahren zur Herstellung einer Verbindung mit der allgemeinen Formel I

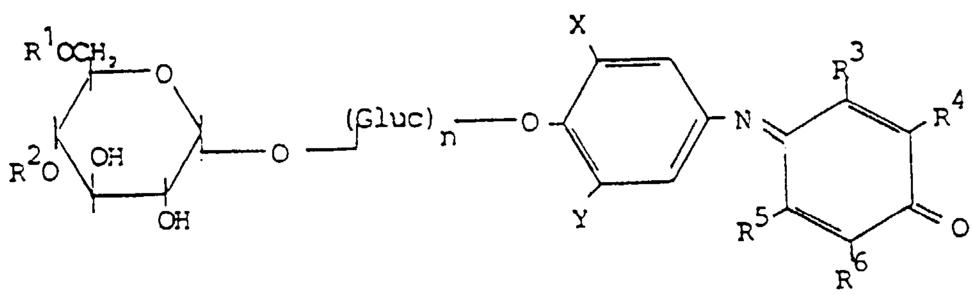

(I)

worin Gluc ein Glucosemolekül ist und der Indophenolrest in α-Stellung mit dem Zucker verknüpft ist,

n = 1 oder 2,
$R^1$ = H, eine geradkettige oder verzweigte Alkyl- oder Alkoylgruppe mit 1 bis 6 C-Atomen, eine Cycloalkyl- oder Cycloalkoylgruppe mit 3 bis 6 C-Atomen, eine Benzoyl-Benzyl- oder Phenylgruppe,
$R^2$ = H,
oder worin $R^1$ und $R^2$ zusammen eine Methylenbrücke bilden, deren H-Atome unabhängig voneinander durch

je eine Alkylgruppe mit 1 bis 5 C-Atomen oder eine Phenylgruppe substituiert sein können,
$R^3$, $R^4$, $R^5$, $R^6$ = unabhängig voneinander H, eine Halogen-(F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe, wobei $R^3$ und $R^4$ oder/und $R^5$ und $R^6$ miteinander verbunden sein und kondensierte Ringe bilden können,
X, Y = unabhängig voneinander H, eine Halogen- (F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe, unter der Vorraussetzung, daß X und Y nicht gleichzeitig H sind,

**dadurch gekennzeichnet,**
daß man

(a) Maltose oder Maltotriose auf an sich bekannte Weise peracetyliert, wobei eine Verbindung mit der allgemeinen Formel II erzeugt wird

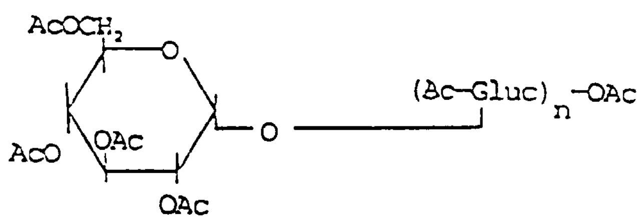

(II)

worin Ac-Gluc eine peracetylierte Glucose bedeutet und der Acetylrest am reduzierenden Ende von II in β-Stellung mit dem Zucker verknüpft ist,
(b) die Verbindung II in Gegenwart eines Säure-Katalysators mit einem substituierten p-Nitrophenol der allgemeinen Formel III umsetzt

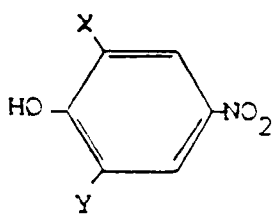

(III)

worin X und Y die zuvor genannten Bedeutungen besitzen, wodurch eine Verbindung mit der allgemeinen Formel IV erzeugt wird,

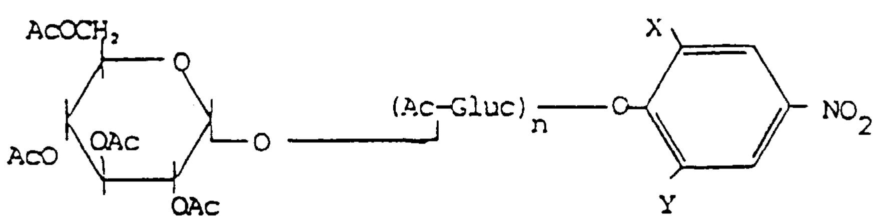

(IV)

worin der p-Nitrophenylrest in α-Stellung mit dem Zucker verknüpft ist,
(c) die Nitrogruppe von IV auf an sich bekannte Weise zu einer Aminogruppe reduziert,
(d) die Acetylreste der aus Schritt (c) resultierenden Verbindung abspaltet,
(e) die aus Schritt (d) resultierende Verbindung mit einem gegebenenfalls substituierten p-Benzochinon der allgemeinen Formel V umsetzt,

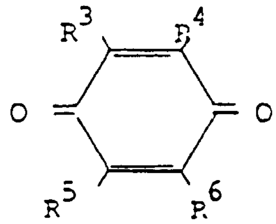

(V)

worin $R^3$, $R^4$, $R^5$ und $R^6$ die zuvor genannten Bedeutungen besitzen, wobei eine Verbindung mit der allgemeinen Formel VI erzeugt wird,

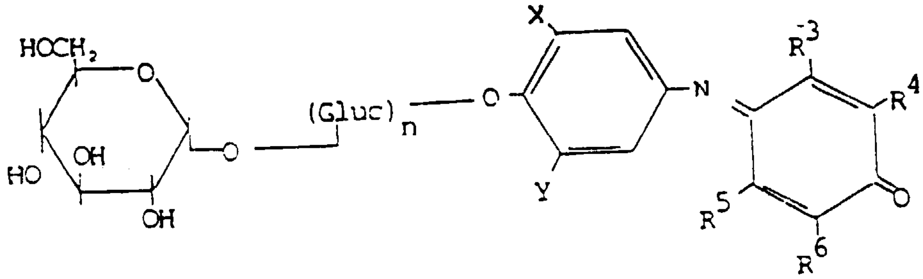

(VI)

worin der Indophenylrest in α-Stellung mit dem Zucker verknüpft ist, und gegebenenfalls
(f) auf an sich bekannte Weise eine Substitution der Positionen 4 und 6 am nicht reduzierenden Ende der Maltose- bzw. Maltotrioserests mit den Resten $R^1$ und $R^2$ einführt, wobei eine Verbindung mit der allgemeinen Formel I erzeugt wird.

2. Verfahren nach Anspruch 1,
**dadurch gekennzeichnet,**
daß $R^1$ und $R^2$ Wasserstoff bedeuten.

3. Verfahren nach Anspruch 1 oder 2,
**dadurch gekennzeichnet,**
daß n = 2 bedeutet.

4. Verfahren nach einem der Ansprüche 1 bis 3,
**dadurch gekennzeichnet,**
daß einer der beiden Reste X und Y Wasserstoff und der andere von Wasserstoff verschieden ist.

5. Verfahren nach einem der Ansprüche 1 bis 4,
**dadurch gekennzeichnet,**
daß mindestens einer der Reste X und Y eine Halogengruppe bedeutet.

6. Verfahren nach Anspruch 5,
**dadurch gekennzeichnet,**
daß die Halogengruppe ein Chlor ist.

7. Verfahren nach einem der Ansprüche 1 bis 6,
**dadurch gekennzeichnet,**
daß $R^3$ und $R^5$ Wasserstoff bedeuten und $R^4$ und $R^6$ Halogen bedeuten.

8. Verfahren nach Anspruch 7,
**dadurch gekennzeichnet,**
daß $R^4$ und $R^6$ Chlor bedeuten.

9. Verfahren nach Anspruch 1,
**dadurch gekennzeichnet,**
daß die Verbindung der Formel I 2', 2, 6-Trichlor-indophenyl-α-maltotriosid ist.

10. Verfahren zur Bestimmung der Aktivität von α-Amylase in wäßriger Lösung, insbesondere in Körperflüssigkeiten

mit einem synthetischen Enzymsubstrat,
**dadurch gekennzeichnet,**
daß man als Enzymsubstrat eine Verbindung mit der allgemeinen Formel I nach Anspruch 1 verwendet.

**11.** Verfahren nach Anspruch 10,
**dadurch gekennzeichnet,**
daß man eine Verbindung I verwendet, in der $R^1$ und $R^2$ Wasserstoff bedeuten.

**12.** Verfahren nach Anspruch 10 oder 11,
**dadurch gekennzeichnet,**
daß man eine Verbindung I verwendet, in der einer der beiden Reste X und Y Wasserstoff und der andere von Wasserstoff verschieden ist.

**13.** Verfahren nach Anspruch 12,
**dadurch gekennzeichnet,**
daß man 2', 2, 6-Trichlor-indophenyl-α-maltotriosid als Enzymsubstrat verwendet.

**14.** Verfahren nach einem der Ansprüche 10 bis 13,
**dadurch gekennzeichnet,**
daß man die Aktivitätsbestimmung von α-Amylase in Abwesenheit von Hilfsenzymen durchführt.

**15.** Verfahren nach einem der Ansprüche 10 bis 14,
**dadurch gekennzeichnet,**
daß man spezifisch die Aktivität von Pankreas-α-Amylase in Gegenwart von Speichel-α-Amylase unter Verwendung eines spezifischen Hemmstoff-Systems für Speichel-α-Amylase bestimmt.

**16.** Verfahren nach Anspruch 15,
**dadurch gekennzeichnet,**
daß man als Hemmstoffsystem einen oder mehrere monoklonale Antikörper verwendet.

**17.** Verwendung einer nach Anspruch 1 hergestellten Verbindung der allgemeinen Formel I als synthetisches Enzymsubstrat in einem Reagenz zur direkten spezifischen Bestimmung von α-Amylase.

**18.** Verwendung nach Anspruch 18,
**dadurch gekennzeichnet,**
daß das Reagenz als Substrat eine Verbindung I enthält, in der $R^1$ und $R^2$ Wasserstoff bedeuten.

**19.** Verwendung nach Anspruch 17 oder 18,
**dadurch gekennzeichnet,**
daß das Reagenz als Substrat eine Verbindung I enthält, in der einer der beiden Reste X und Y Wasserstoff und der andere von Wasserstoff verschieden ist.

**20.** Verwendung nach Anspruch 19,
**dadurch gekennzeichnet,**
daß das Reagenz als Substrat 2', 2, 6-Trichlor-indophenyl-α-maltotriosid enthält.

**21.** Verwendung nach einem der Ansprüche 17 bis 20,
**dadurch gekennzeichnet,**
daß das Reagenz zusätzlich einen Aktivator enthält.

**22.** Verwendung nach Anspruch 21,
**dadurch gekennzeichnet,**
daß der Aktivator ein Azid- oder ein Rhodanidsalz ist.

**23.** Verwendung nach einem der Ansprüche 17 bis 22,
**dadurch gekennzeichnet,**
daß das Reagenz ein spezifisches Hemmstoff-System für Speichel-α-Amylase enthält.

**24.** Verwendung nach Anspruch 23,
**dadurch gekennzeichnet,**
daß das Hemmstoff-System aus einem oder mehreren monoklonalen Antikörpern besteht.

**25.** Verwendung nach Anspruch 17 zur direkten Bestimmung der enzymatischen Aktivität von α-Amylase.

## Claims

**Claims for the following Contracting States : AT, BE, CH, LI, DE, DK, FR, GB, IT, LU, NL, SE**

**1.** Compound having the general formula I

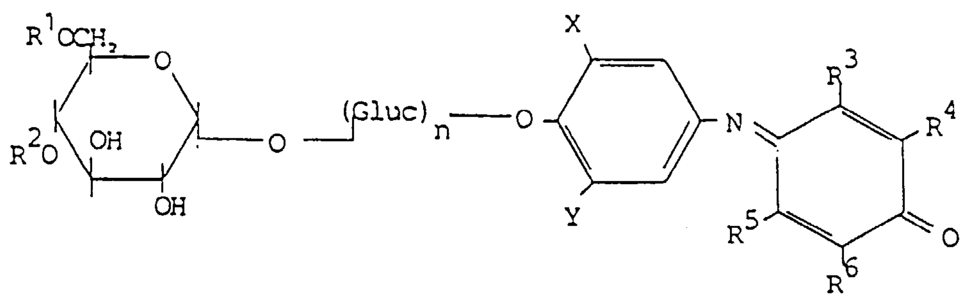

(I)

in which
Gluc is a glucose molecule and the indophenol residue is linked to the sugar in the α-position,

n = 1 or 2,
$R^1$ = H, a straight-chained or branched alkyl or alkoyl group with 1 to 6 C atoms, a cycloalkyl or cycloalkoyl group with 3 to 6 C atoms, a benzoyl, benzyl or phenyl group,
$R^2$ = H,
or in which $R^1$ and $R^2$ together form a methylene bridge, each of the H atoms of which can be independently substituted by an alkyl group with 1 to 5 C atoms or a phenyl group,
$R^3$, $R^4$, $R^5$, $R^6$ are independently of one another H, a halogen (F, Cl, Br), nitro, acetamide or cyano group, a sulfonic acid group, sulfinic acid group or carboxylic acid group as well as the corresponding acid alkyl ester groups and acid amide groups, an alkoxy group, a straight-chained or branched alkyl group with 1 to 6 C atoms which is substituted if desired, a cycloalkyl group with 3 to 6 C atoms which is substituted if desired or an aryl group which is substituted if desired, whereby $R^3$ and $R^4$ or/and $R^5$ and $R^6$ can be linked to one another and can form condensed rings,
X, Y are independently of one another H, a halogen (F, Cl, Br), nitro, acetamide or cyano group, a sulfonic acid, sulfinic acid or carboxylic acid group as well as the corresponding acid alkyl ester and acid amide groups, an alkoxy group, a straight-chained or branched alkyl group with 1 to 6 C atoms which is substituted if desired, a cycloalkyl group with 3 to 6 C atoms which is substituted if desired or an aryl group which is substituted if desired, provided that X and Y are not simultaneously H.

**2.** Compound as claimed in claim 1,
**wherein**
$R^1$ and $R^2$ denote hydrogen.

**3.** Compound as claimed in claim 1 or 2,
**wherein**
n = 2.

**4.** Compound as claimed in one of the claims 1 to 3,
**wherein**
one of the two residues X and Y is hydrogen and the other is different from hydrogen.

**5.** Compound as claimed in one of the claims 1 to 4,

**wherein**
at least one of the residues X and Y denotes a halogen group.

6. Compound as claimed in claim 5,
**wherein**
the halogen group is chlorine.

7. Compound as claimed in one of the claims 1 to 6,
**wherein**
$R^3$ and $R^5$ denote hydrogen and $R^4$ and $R^6$ denote halogen.

8. Compound as claimed in claim 7,
**wherein**
$R^4$ and $R^6$ denote chlorine.

9. 2',2,6-trichloro-indophenyl-α-maltotrioside.

10. Process for the production of a compound having the general formula I as claimed in claim 1,
**wherein**

(a) maltose or maltotriose is peracetylated in a known way to form a compound having the general formula II

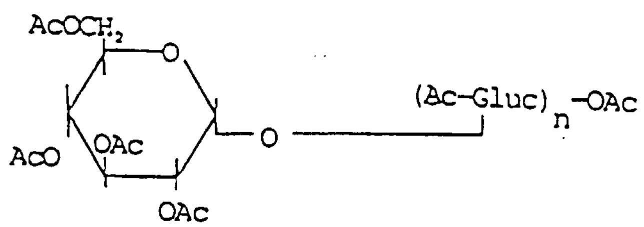

(II)

in which Ac-Gluc denotes a peracetylated glucose and the acetyl residue at the reducing end of II is linked in the β-position to the sugar,
(b) the compound II is reacted in the presence of an acid catalyst with a substituted p-nitrophenol of the general formula III

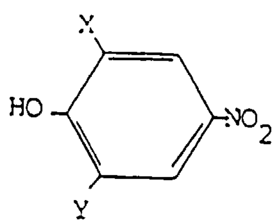

(III)

in which X and Y have the meanings stated in claim 1, whereupon a compound is formed having the general formula IV,

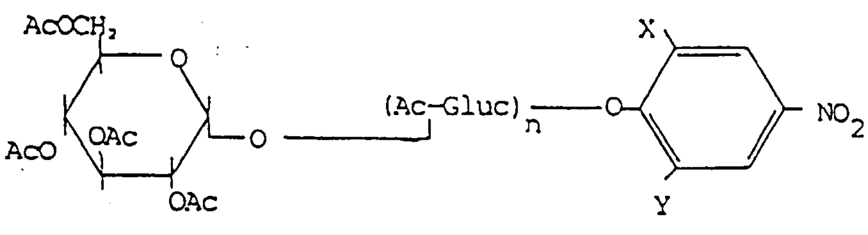

(IV)

in which the p-nitrophenyl residue is linked to the sugar in the α-position,
(c) the nitro group of IV is reduced to form an amino group in a known manner,
(d) the acetyl residues of the compound resulting from step (c) are cleaved off,
(e) the compound resulting from step (d) is reacted with a p-benzoquinone which is substituted if desired having the general formula V,

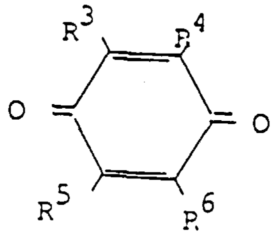

(V)

in which $R^3$, $R^4$, $R^5$ and $R^6$ have the meanings stated in claim 1, to form a compound having the general formula VI,

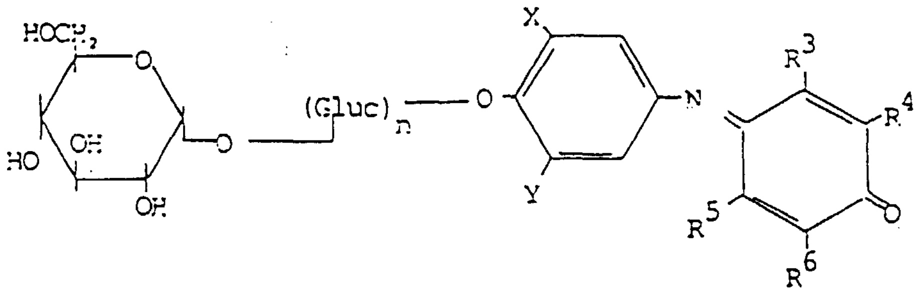

(VI)

in which the indophenyl residue is linked to the sugar in the α-position and if desired

(f) a substitution of positions 4 and 6 at the non-reducing end of the maltose or maltotriose residue by the residues $R^1$ and $R^2$ is introduced in a known way to form a compound having the general formula I,

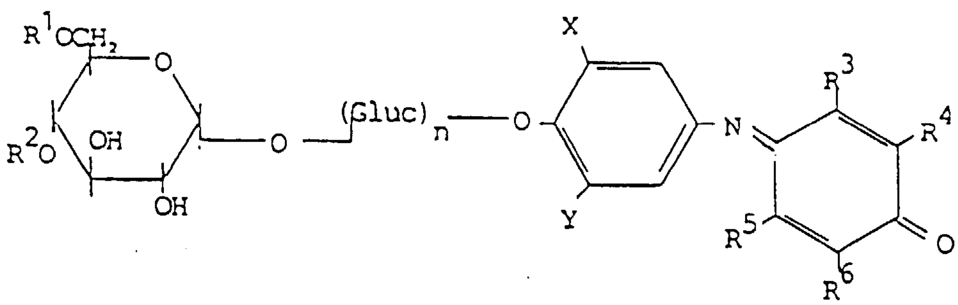

(I)

in which all the symbols have the meanings stated in claim 1.

**11.** Method for the determination of the activity of α-amylase in an aqueous solution, in particular in body fluids, with a synthetic enzyme substrate,
**wherein**
a compound having the general formula I as claimed in claim 1 is used as the enzyme substrate.

**12.** Method as claimed in claim 11,
**wherein**
a compound I is used in which $R^1$ and $R^2$ denote hydrogen.

**13.** Method as claimed in claim 11 or 12,
**wherein**
a compound I is used in which one of the two residues X and Y is hydrogen and the other is different from hydrogen.

**14.** Method as claimed in claim 13,
**wherein**
2',2,6-trichloro-indophenyl-α-maltotrioside is used as the enzyme substrate.

**15.** Method as claimed in one of the claims 11 to 14,
**wherein**
the determination of the α-amylase activity is carried out in the absence of auxiliary enzymes.

**16.** Method as claimed in one of the claims 11 to 15,
**wherein**
the activity of pancreatic α-amylase is determined specifically in the presence of salivary α-amylase using a specific inhibitor system for salivary α-amylase.

**17.** Method as claimed in claim 16,
**wherein**
one or several monoclonal antibodies are used as the inhibitor system.

**18.** Reagent for the direct specific determination of α-amylase with a synthetic enzyme substrate,
**wherein**
it contains a compound having the general formula I as claimed in claim 1 as the enzyme substrate.

**19.** Reagent as claimed in claim 18,
**wherein**
it contains a compound I as the substrate in which $R^1$ and $R^2$ denote hydrogen.

**20.** Reagent as claimed in claim 18 or 19,
**wherein**
it contains a compound I as the substrate in which one of the two residues X and Y is hydrogen and the other is different from hydrogen.

**21.** Reagent as claimed in claim 20,
**wherein**
it contains 2',2,6-trichloro-indophenyl-α-maltotrioside as the substrate.

**22.** Reagent as claimed in one of the claims 18 to 21,
**wherein**
it in addition contains an activator.

**23.** Reagent as claimed in claim 22,
**wherein**
the activator is an azide salt or a thiocyanate salt.

**24.** Reagent as claimed in one of the claims 18 to 23,
**wherein**
it contains a specific inhibitor system for salivary α-amylase.

**25.** Reagent as claimed in claim 24,
**wherein**
the inhibitor system consists of one or several monoclonal antibodies.

**26.** Use of a compound as claimed in one of the claims 1 to 9 as the synthetic enzyme substrate.

**27.** Use as claimed in claim 26 for the direct determination of the enzymatic activity of α-amylase.

**Claims for the following Contracting States : ES, GR**

**1.** Process for the production of a compound having the general formula I

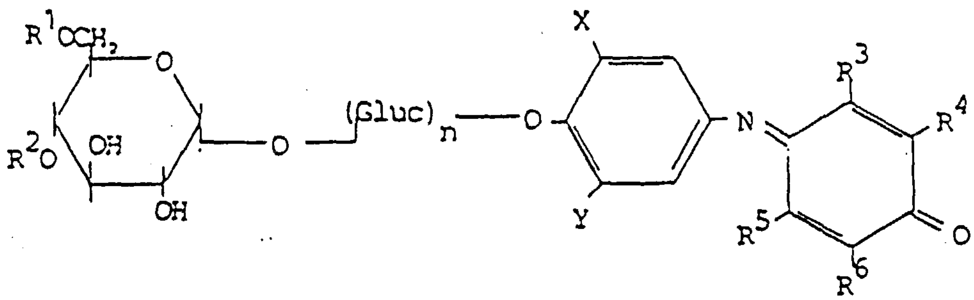

(I)

in which Gluc is a glucose molecule and the indophenol residue is linked to the sugar in the α-position,

n = 1 or 2,
$R^1$ = H, a straight-chained or branched alkyl or alkoyl group with 1 to 6 C atoms, a cycloalkyl or cycloalkoyl group with 3 to 6 C atoms, a benzoyl, benzyl or phenyl group,
$R^2$ = H,
or in which $R^1$ and $R^2$ together form a methylene bridge, each of the H atoms of which can be independently substituted by an alkyl group with 1 to 5 C atoms or a phenyl group,
$R^3$, $R^4$, $R^5$, $R^6$ are independently of one another H, a halogen (F, Cl, Br), nitro, acetamide or cyano group, a sulfonic acid group, sulfinic acid group or carboxylic acid group as well as the corresponding acid alkyl ester groups and acid amide groups, an alkoxy group, a straight-chained or branched alkyl group with 1 to 6 C atoms which is substituted if desired, a cycloalkyl group with 3 to 6 C atoms which is substituted if desired or an aryl group which is substituted if desired, whereby $R^3$ and $R^4$ or/and $R^5$ and $R^6$ can be linked to one another and can form condensed rings,
X, Y are independently of one another H, a halogen (F, Cl, Br), nitro, acetamide or cyano group, a sulfonic acid, sulfinic acid or carboxylic acid group as well as the corresponding acid alkyl ester and acid amide groups, an alkoxy group, a straight-chained or branched alkyl group with 1 to 6 C atoms which is substituted if desired, a cycloalkyl group with 3 to 6 C atoms which is substituted if desired or an aryl group which is substituted if desired, provided that X and Y are not simultaneously H,

**wherein**

(a) maltose or maltotriose is peracetylated in a known way to form a compound having the general formula II

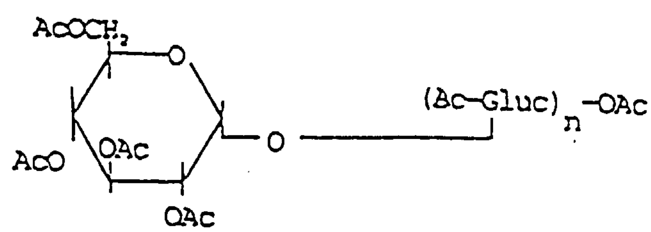

(II)

in which Ac-Gluc denotes a peracetylated glucose and the acetyl residue at the reducing end of II is linked in the β-position to the sugar,
(b) the compound II is reacted in the presence of an acid catalyst with a substituted p-nitrophenol of the general formula III

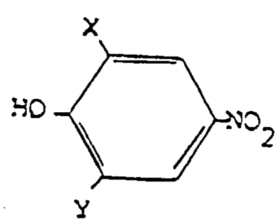

(III)

in which X and Y have the meanings stated above, whereupon a compound is formed having the general formula IV,

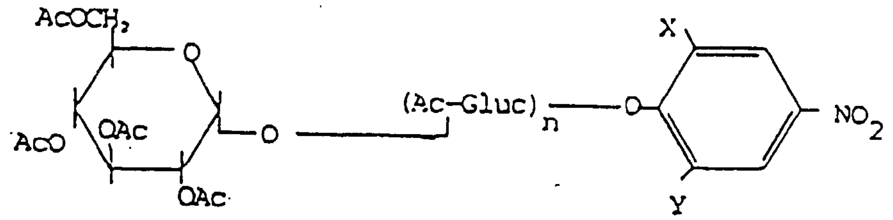

(IV)

in which the p-nitrophenyl residue is linked to the sugar in the α-position,
(c) the nitro group of IV is reduced to form an amino group in a known manner,
(d) the acetyl residues of the compound resulting from step (c) are cleaved off,
(e) the compound resulting from step (d) is reacted with a p-benzoquinone which is substituted if desired having the general formula V,

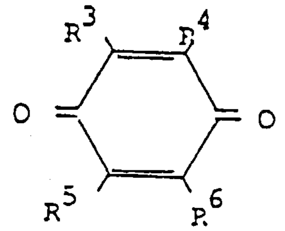

(V)

in which $R^3$, $R^4$, $R^5$ and $R^6$ have the meanings stated above to form a compound having the general formula VI,

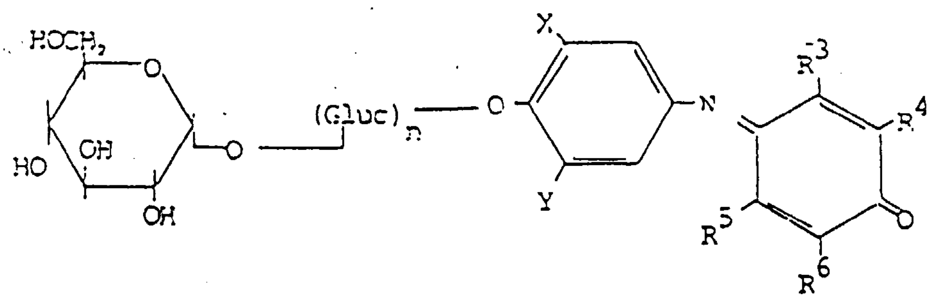

(VI)

in which the indophenyl residue is linked to the sugar in the α-position and if desired
(f) a substitution of positions 4 and 6 at the non-reducing end of the maltose or maltotriose residue by the residues $R^1$ and $R^2$ is introduced in a known way to form a compound having the general formula I.

**2.** Process as claimed in claim 1,
**wherein**
$R^1$ and $R^2$ denote hydrogen.

**3.** Process as claimed in claim 1 or 2,
**wherein**
n = 2.

**4.** Process as claimed in one of the claims 1 to 3,
**wherein**
one of the two residues X and Y is hydrogen and the other is different from hydrogen.

**5.** Process as claimed in one of the claims 1 to 4,
**wherein**
at least one of the residues X and Y denotes a halogen group.

**6.** Process as claimed in claim 5,
**wherein**
the halogen group is chlorine.

**7.** Process as claimed in one of the claims 1 to 6,

**wherein**
$R^3$ and $R^5$ denote hydrogen and $R^4$ and $R^6$ denote halogen.

**8.** Process as claimed in claim 7,
**wherein**
$R^4$ and $R^6$ denote chlorine.

**9.** Process as claimed in claim 1,
**wherein**
the compound of formula I is 2',2,6-trichloroindophenyl-α-maltotrioside.

**10.** Method for the determination of the activity of α-amylase in an aqueous solution in particular in body fluids with a synthetic enzyme substrate,
**wherein**
a compound having the general formula I is used as the enzyme substrate as claimed in claim 1.

**11.** Method as claimed in claim 10,
**wherein**
a compound I is used in which $R^1$ and $R^2$ denote hydrogen.

**12.** Method as claimed in claim 10 or 11,
**wherein**
a compound I is used in which one of the two residues X and Y is hydrogen and the other is different from hydrogen.

**13.** Method as claimed in claim 12,
**wherein**
2', 2,6-trichloro-indophenyl-α-maltotrioside is used as the enzyme substrate.

**14.** Method as claimed in one of the claims 10 to 13,
**wherein**
the determination of the α-amylase activity is carried out in the absence of auxiliary enzymes.

**15.** Method as claimed in one of the claims 10 to 14,
**wherein**
the activity of pancreatic α-amylase is determined specifically in the presence of salivary α-amylase using a specific inhibitor system for salivary α-amylase.

**16.** Method as claimed in claim 15,
**wherein**
one or several monoclonal antibodies are used as the inhibitor system.

**17.** Use of a compound having the general formula I as claimed in claim 1 as the synthetic enzyme substrate in a reagent for the direct specific determination of α-amylase.

**18.** Use as claimed in claim 18,
**wherein**
the reagent contains a compound I as the substrate in which $R^1$ and $R^2$ denote hydrogen.

**19.** Use as claimed in claim 17 or 18,
**wherein**
the reagent contains a compound I as the substrate in which one of the two residues X and Y is hydrogen and the other is different from hydrogen.

**20.** Use as claimed in claim 19,
**wherein**
the reagent contains 2',2,6-trichloro-indophenyl-α-maltotrioside as the substrate.

**21.** Use as claimed in one of the claims 17 to 20,

**wherein**
the reagent additionally contains an activator

**22.** Use as claimed in claim 21,
**wherein**
the activator is an azide salt or a thiocyanate salt.

**23.** Use as claimed in one of the claims 17 to 22,
**wherein**
the reagent contains a specific inhibitor system for salivary α-amylase.

**24.** Use as claimed in claim 23,
**wherein**
the inhibitor system consists of one or several monoclonal antibodies.

**25.** Use as claimed in claim 17 for the direct determination of the enzymatic activity of α-amylase.

**Revendications**

**Revendications pour les Etas contractants suivants : AT, BE, CH, LI, DE, DK, FR, GB, IT, LU, NL, SE**

**1.** Composé de formule générale (I)

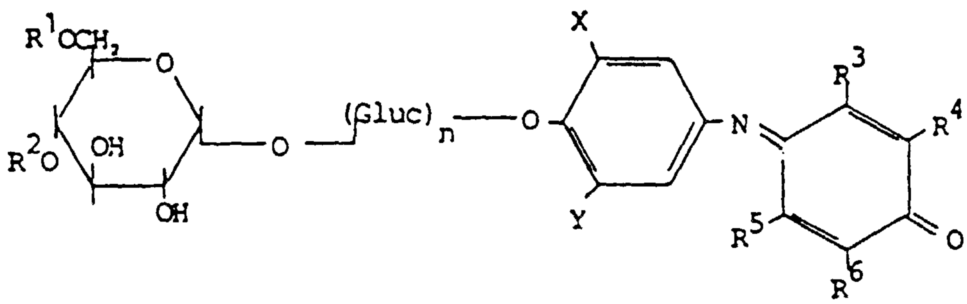

(I)

dans laquelle
Gluc est une molécule de glucose et le reste indophénol est lié au sucre en position α,

n = 1 ou 2,
$R^1$ = H, un groupe alkyle ou alcoyle linéaire ou ramifié de 1 à 6 atomes de carbone, un groupe cycloalkyle ou cycloalcoyle de 3 à 6 atomes de carbone, un groupe benzoyle, benzyle ou phényle,
$R^2$ = H,
ou dans laquelle $R^1$ et $R^2$ forment ensemble un pont méthylène dont les atomes d'hydrogène peuvent être substitués indépendamment l'un de l'autre chacun par un groupe alkyle de 1 à 5 atomes de carbone ou par un groupe phényle,
$R^3$, $R^4$, $R^5$, $R^6$ = indépendamment les uns des autres H, un groupe halogène (F, Cl, Br), nitro, acétamide ou cyano, un groupe acide sulfonique, acide sulfinique ou acide carboxylique et les groupes alkylesters d'acide et amides d'acide correspondants, un groupe alcoxy, un groupe alkyle linéaire ou ramifié de 1 à 6 atomes de carbone éventuellement substitué, un groupe cycloalkyle de 3 à 6 atomes de carbone éventuellement substitué ou un groupe aryle éventuellement substitué, $R^3$ et $R^4$ et/ou $R^5$ et $R^6$ pouvant être liés entre eux et former des cycles condensés,
X, Y = indépendamment l'un de l'autre H, un groupe halogène (F, Cl, Br), nitro, acétamide ou cyano, un groupe acide sulfonique, acide sulfinique ou acide carboxylique et les groupes alkylesters d'acide et amides d'acide correspondants, un groupe alcoxy, un groupe alkyle linéaire ou ramifié de 1 à 6 atomes de carbone éventuellement substitué, un groupe cycloalkyle de 3 à 6 atomes de carbone éventuellement substitué ou un groupe aryle éventuellement substitué,

à condition que X et Y ne soient pas simultanément H.

**2.** Composé selon la revendication 1, caractérisé en ce que $R^1$ et $R^2$ représentent l'hydrogène.

**3.** Composé selon la revendication 1 ou 2, caractérisé en ce que n = 2.

**4.** Composé selon l'une des revendications 1 à 3, caractérisé en ce que l'un des deux restes X et Y représente l'hydrogène et l'autre est différent de l'hydrogène.

**5.** Composé selon l'une des revendications 1 à 4, caractérisé en ce qu'au moins l'un des restes X et Y représente un groupe halogène.

**6.** Composé selon la revendication 5, caractérisé en ce que le groupe halogène est le chlore.

**7.** Composé selon l'une des revendications 1 à 6, caractérisé en ce que $R^3$ et $R^5$ représentent l'hydrogène et $R^4$ et $R^6$ représentent un halogène.

**8.** Composé selon la revendication 7, caractérisé en ce que $R^4$ et $R^6$ représentent le chlore.

**9.** 2',2,6-trichloroindophényl-α-maltotrioside.

**10.** Procédé de préparation d'un composé de formule générale I selon la revendication 1, caractérisé en ce que

(a) on peracétyle de manière connue en soi du maltose ou du maltotriose de sorte qu'il se forme un composé de formule générale II

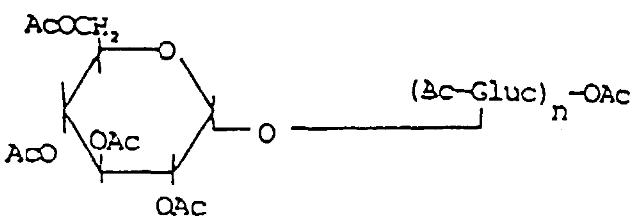

(II)

dans laquelle Ac-Gluc représente un glucose peracétylé et le reste acétyle à l'extrémité réductrice de II est lié au sucre en position β,

(b) on fait réagir le composé II en présence d'un catalyseur acide avec un p-nitrophénol substitué de formule générale III

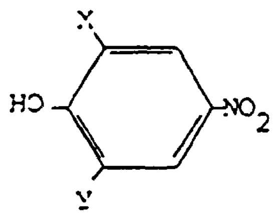

(III)

dans laquelle X et Y possèdent les significations citées dans la revendication 1, de sorte qu'il se forme un composé de formule générale IV

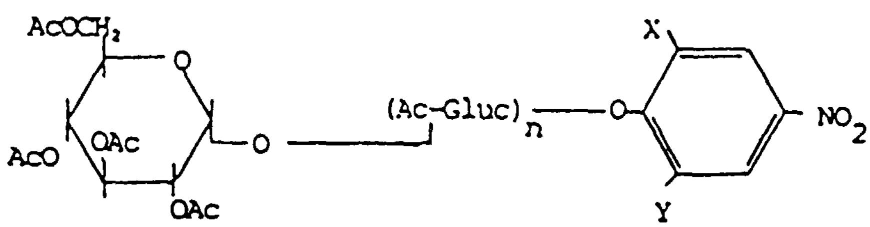

(IV)

dans laquelle le reste p-nitrophényle est lié au sucre en position α,

(c) on réduit de manière connue en soi le groupe nitro de IV en un groupe amino,

(d) on clive les restes acétyle du composé résultant de l'étape (c),

(e) on fait réagir le composé résultant de l'étape (d) avec une p-benzoquinone éventuellement substituée de formule générale v

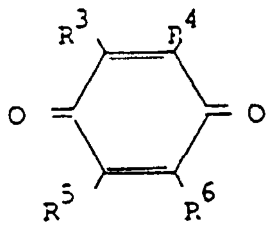

(V)

dans laquelle $R^3$, $R^4$, $R^5$ et $R^6$ possèdent les significations citées dans la revendication 1, de sorte qu'il se forme un composé de formule générale VI

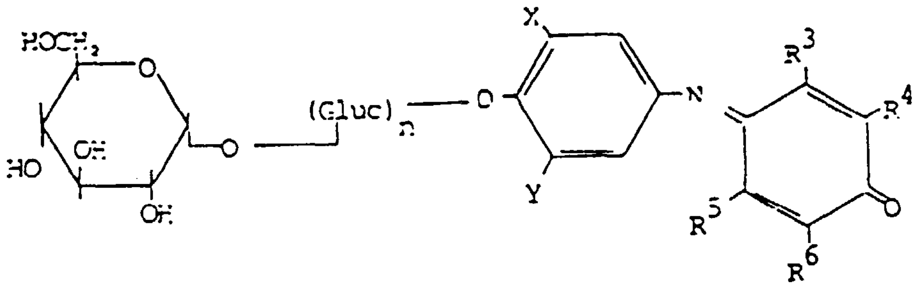

(VI)

dans laquelle le reste indophényle est lié au sucre en position α, et éventuellement

(f) on introduit de manière connue en soi une substitution des positions 4 et 6 à l'extrémité non réductrice du reste maltose ou maltotriose avec les restes $R^1$ et $R^2$, de sorte qu'il se forme un composé de formule générale I

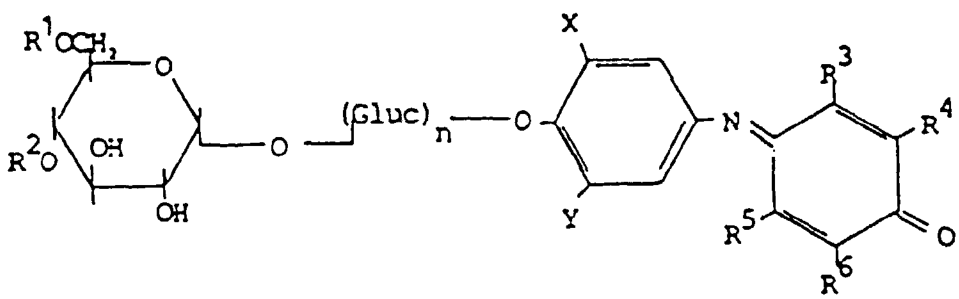

(I)

dans laquelle tous les symboles possèdent les significations citées dans la revendication 1.

**11.** Procédé pour déterminer l'activité de l'α-amylase en solution aqueuse, en particulier dans les liquides de l'organisme avec un substrat d'enzyme synthétique, caractérisé en ce que l'on utilise comme substrat d'enzyme un composé de formule générale I selon la revendication 1.

**12.** Procédé selon la revendication 11, caractérisé en ce que l'on utilise un composé I dans lequel $R^1$ et $R^2$ représentent l'hydrogène.

**13.** Procédé selon la revendication 11 ou 12, caractérisé en ce que l'on utilise un composé I dans lequel l'un des deux restes X et Y est l'hydrogène et l'autre est différent de l'hydrogène.

**14.** Procédé selon la revendication 13, caractérisé en ce que l'on utilise le 2',2,6-trichloroindophényl-α-maltotrioside comme substrat d'enzyme.

**15.** Procédé selon l'une des revendications 11 à 14, caractérisé en ce que l'on réalise la détermination de l'activité de l'α-amylase en l'absence d'enzymes auxiliaires.

**16.** Procédé selon l'une des revendications 11 à 15, caractérisé en ce que l'on détermine spécifiquement l'activité de l'α-amylase pancréatique en présence d'α-amylase salivaire en utilisant un système inhibiteur spécifique pour l'α-amylase salivaire.

**17.** Procédé selon la revendication 16, caractérisé en ce que l'on utilise comme système inhibiteur un ou plusieurs anticorps monoclonaux.

**18.** Réactif pour la détermination spécifique directe de l'α-amylase avec un substrat d'enzyme synthétique, caractérisé en ce qu'il contient comme substrat d'enzyme un composé de formule générale I selon la revendication 1.

**19.** Réactif selon la revendication 18, caractérisé en ce qu'il contient comme substrat un composé I dans lequel $R^1$ et $R^2$ représentent l'hydrogène.

**20.** Réactif selon la revendication 18 ou 19, caractérisé en ce qu'il contient comme substrat un composé I dans lequel l'un des deux restes X et Y est l'hydrogène et l'autre est différent de l'hydrogène.

**21.** Réactif selon la revendication 20, caractérisé en ce qu'il contient comme substrat le 2',2,6-trichloroindophényl-α-maltotrioside.

**22.** Réactif selon l'une des revendications 18 à 21, caractérisé en ce qu'il contient en outre un activateur.

**23.** Réactif selon la revendication 22, caractérisé en ce que l'activateur est un sel d'azide ou de rhodanide.

**24.** Réactif selon l'une des revendications 18 à 23, caractérisé en ce qu'il contient un système inhibiteur spécifique pour l'α-amylase salivaire.

**25.** Réactif selon la revendication 24, caractérisé en ce que le système inhibiteur consiste en un ou plusieurs anticorps monoclonaux.

**26.** Utilisation d'un composé selon l'une des revendications 1 à 9 comme substrat d'enzyme synthétique.

**27.** Utilisation selon la revendication 26 pour la détermination directe de l'activité enzymatique de l'α-amylase.

**Revendications pour les Etats contractants suivants : ES, GR**

**1.** Procédé de préparation d'un composé de formule générale I

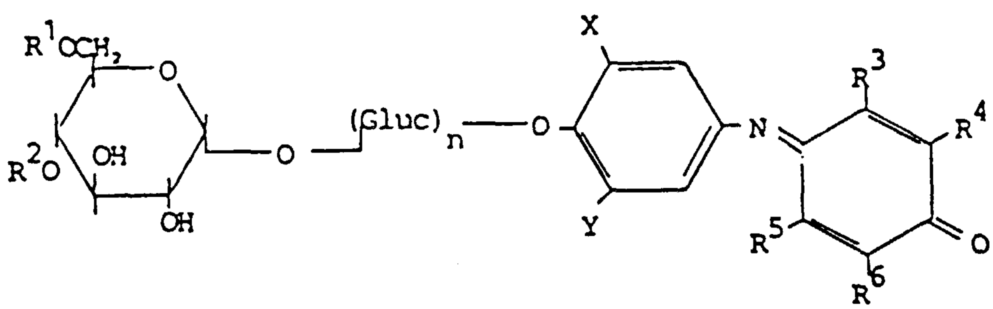

(I)

dans laquelle
Gluc est une molécule de glucose et le reste indophénol est lié au sucre en position α,

n = 1 ou 2,
$R^1$ = H, un groupe alkyle ou alcoyle linéaire ou ramifié de 1 à 6 atomes de carbone, un groupe cycloalkyle ou cycloalcoyle de 3 à 6 atomes de carbone, un groupe benzoyle, benzyle ou phényle,
$R^2$ = H,
ou dans laquelle $R^1$ et $R^2$ forment ensemble un pont méthylène dont les atomes d'hydrogène peuvent être substitués indépendamment l'un de l'autre chacun par un groupe alkyle de 1 à 5 atomes de carbone ou par un groupe phényle,
$R^3$, $R^4$, $R^5$, $R^6$ = indépendamment les uns des autres H, un groupe halogène (F, Cl, Br), nitro, acétamide ou cyano, un groupe acide sulfonique, acide sulfinique ou acide carboxylique et les groupes alkylesters d'acide et amides d'acide correspondants, un groupe alcoxy, un groupe alkyle linéaire ou ramifié de 1 à 6 atomes de carbone éventuellement substitué, un groupe cycloalkyle de 3 à 6 atomes de carbone éventuellement substitué ou un groupe aryle éventuellement substitué, $R^3$ et $R^4$ et/ou $R^5$ et $R^6$ pouvant être liés entre eux et former des cycles condensés,
X, Y = indépendamment l'un de l'autre H, un groupe halogène (F, Cl, Br), nitro, acétamide ou cyano, un groupe

acide sulfonique, acide sulfinique ou acide carboxylique et les groupes alkylesters d'acide et amides d'acide correspondants, un groupe alcoxy, un groupe alkyle linéaire ou ramifié de 1 à 6 atomes de carbone éventuellement substitué, un groupe cycloalkyle de 3 à 6 atomes de carbone éventuellement substitué ou un groupe aryle éventuellement substitué, à condition que X et Y ne soient pas simultanément H, caractérisé en ce que

(a) on peracétyle de manière connue en soi du maltose ou du maltotriose de sorte qu'il se forme un composé de formule générale II

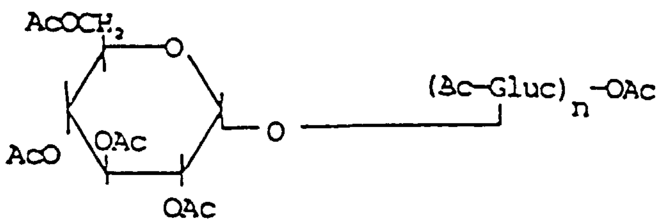

(II)

dans laquelle Ac-Gluc représente un glucose peracétylé et le reste acétyle à l'extrémité réductrice de II est lié au sucre en position β,
(b) on fait réagir le composé II en présence d'un catalyseur acide avec un p-nitrophénol substitué de formule générale III

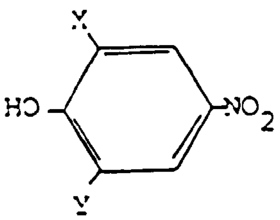

(III)

dans laquelle X et Y possèdent les significations citées précédemment, de sorte qu'il se forme un composé de formule générale IV

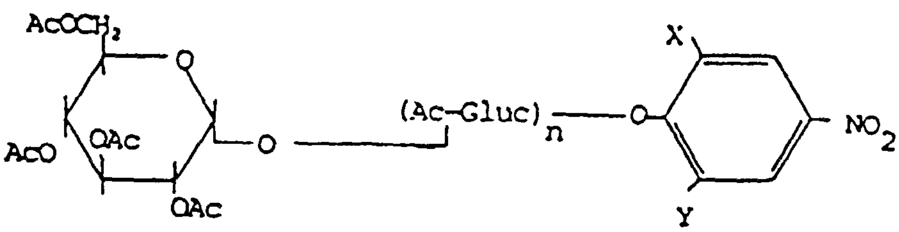

(IV)

dans laquelle le reste p-nitrophényle est lié au sucre en position α,
(c) on réduit de manière connue en soi le groupe nitro de IV en un groupe amino,
(d) on clive les restes acétyle du composé résultant de l'étape (c),
(e) on fait réagir le composé résultant de l'étape (d) avec une p-benzoquinone éventuellement substituée de formule générale V

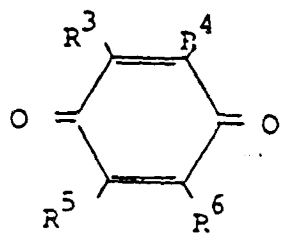

(V)

dans laquelle $R^3$, $R^4$, $R^5$ et $R^6$ possèdent les significations citées précédemment, de sorte qu'il se forme un composé de formule générale VI

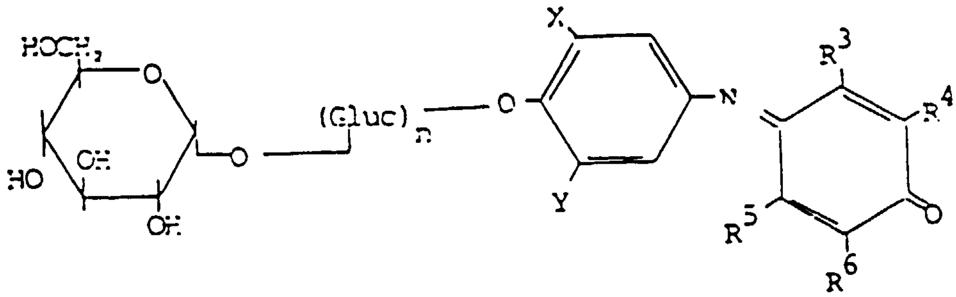

(VI)

dans laquelle le reste indophényle est lié au sucre en position α, et éventuellement

(f) on introduit de manière connue en soi une substitution des positions 4 et 6 à l'extrémité non réductrice du reste maltose ou maltotriose avec les restes $R^1$ et $R^2$, de sorte qu'il se forme un composé de formule générale I.

**2.** Procédé selon la revendication 1, caractérisé en ce que $R^1$ et $R^2$ représentent l'hydrogène.

**3.** Procédé selon la revendication 1 ou 2, caractérisé en ce que n = 2.

**4.** Procédé selon l'une des revendications 1 à 3, caractérisé en ce que l'un des deux restes X et Y représente l'hydrogène et l'autre est différent de l'hydrogène.

**5.** Procédé selon l'une des revendications 1 à 4, caractérisé en ce qu'au moins l'un des restes X et Y représente un groupe halogène.

**6.** Procédé selon la revendication 5, caractérisé en ce que le groupe halogène est un chlore.

**7.** Procédé selon l'une des revendications 1 à 6, caractérisé en ce que $R^3$ et $R^5$ représentent l'hydrogène et $R^4$ et $R^6$ représentent un halogène.

**8.** Procédé selon la revendication 7, caractérisé en ce que $R^4$ et $R^6$ représentent le chlore.

**9.** Procédé selon la revendication 1, caractérisé en ce que le composé de formule I est le 2',2,6-trichloroindophényl-α-maltotrioside.

**10.** Procédé pour déterminer l'activité de l'α-amylase en solution aqueuse, en particulier dans les liquides de l'organisme avec un substrat d'enzyme synthétique, caractérisé en ce que l'on utilise comme substrat d'enzyme un composé de formule générale I selon la revendication 1.

**11.** Procédé selon la revendication 10, caractérisé en ce que l'on utilise un composé I dans lequel $R^1$ et $R^2$ représentent l'hydrogène.

**12.** Procédé selon la revendication 10 ou 11, caractérisé en ce que l'on utilise un composé I dans lequel l'un des deux restes X et Y est l'hydrogène et l'autre est différent de l'hydrogène.

**13.** Procédé selon la revendication 12, caractérisé en ce que l'on utilise le 2',2,6-trichloroindophényl-α-maltotrioside comme substrat d'enzyme.

**14.** Procédé selon l'une des revendications 10 à 13, caractérisé en ce que l'on réalise la détermination de l'activité de l'α-amylase en l'absence d'enzymes auxiliaires.

**15.** Procédé selon l'une des revendications 10 à 14, caractérisé en ce que l'on détermine spécifiquement l'activité de l'α-amylase pancréatique en présence d'α-amylase salivaire en utilisant un système inhibiteur spécifique pour l'α-amylase salivaire.

**16.** Procédé selon la revendication 15, caractérisé en ce que l'on utilise comme système inhibiteur un ou plusieurs anticorps monoclonaux.

**17.** Utilisation d'un composé de formule générale I préparé selon la revendication 1 comme substrat d'enzyme synthétique dans un réactif pour la détermination spécifique directe de l'α-amylase.

**18.** Utilisation selon la revendication 18, caractérisée en ce que le réactif contient comme substrat un composé I dans lequel $R^1$ et $R^2$ représentent l'hydrogène.

**19.** Utilisation selon la revendication 17 ou 18, caractérisée en ce que le réactif contient comme substrat un composé I dans lequel l'un des deux restes X et Y est l'hydrogène et l'autre est différent de l'hydrogène.

**20.** Utilisation selon la revendication 19, caractérisée en ce que le réactif contient comme substrat le 2',2,6-trichloroindophényl-α-maltotrioside.

**21.** Utilisation selon l'une des revendications 17 à 20, caractérisée en ce que le réactif contient en outre un activateur.

**22.** Utilisation selon la revendication 21, caractérisée en ce que l'activateur est un sel d'azide ou de rhodanide.

**23.** Utilisation selon l'une des revendications 17 à 22, caractérisée en ce que le réactif contient un système inhibiteur spécifique pour l'α-amylase salivaire.

**24.** Utilisation selon la revendication 23, caractérisée en ce que le système inhibiteur consiste en un ou plusieurs anticorps monoclonaux.

**25.** Utilisation selon la revendication 17 pour la détermination directe de l'activité enzymatique de l'α-amylase.

Fig. 1

2',2,6-TRICHLORINDOPHENYL-
ALPHA - MALTOTRIOSID:

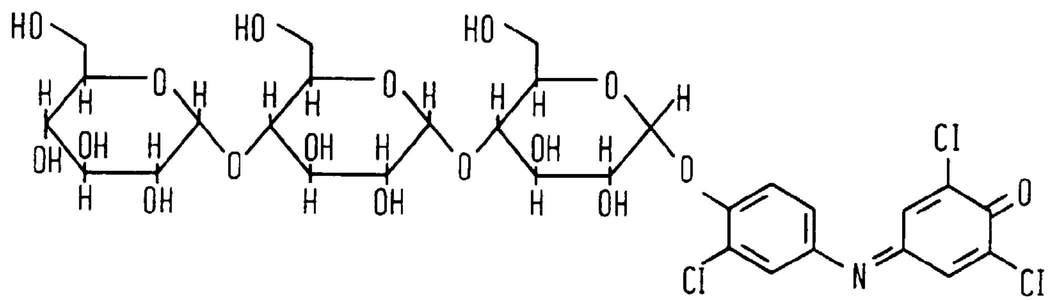

2,6-DICHLORINDOPHENYL-
ALPHA - MALTOTRIOSID:

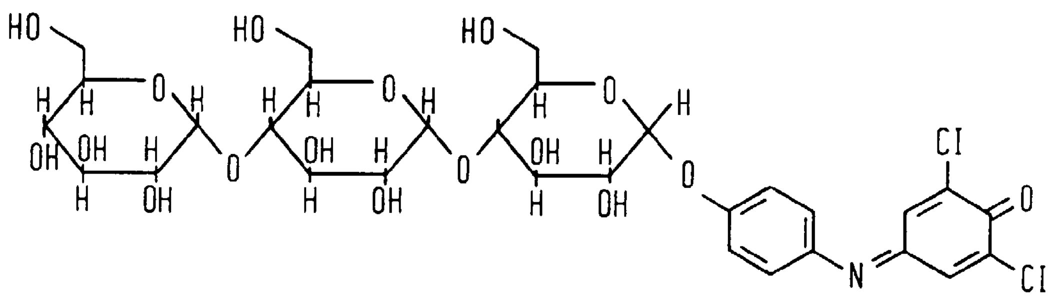

2,6-DIMETHYLINDOPHENYL-
ALPHA - MALTOTRIOSID: